# EUROPÄISCHE PATENTANMELDUNG

(11) **EP 3 666 259 A1**
(43) Veröffentlichungstag der Anmeldung: **17.06.2020**
(21) Anmeldenummer: 19214098.6
(22) Anmeldetag: 06.12.2019
(51) Int. Cl.: A61K 9/12, A61K 8/04, A61K 8/65, A61K 8/73, A61K 8/60, A61Q 7/00, A61Q 19/08, A61K 47/36, A61K 47/42, A61L 15/28, A61L 15/32, B05B 7/04

(54) **VERSCHÄUMBARE WÄSSRIGE ZUBEREITUNGEN AUF BIOPOLYMERBASIS MIT EINSATZFELXIBLER GAS-SPEICHERZELLEN-VERTEILUNG**

(30) Priorität: 13.12.2018 DE 102018009781
(71) Anmelder: Valeopharm GmbH, 22339 Hamburg (DE)
(72) Erfinder: Teslenko, Alexander, 58095 Hagen (DE)
(74) Vertreter: Müller, Claudia

(57) **Zusammenfassung**

Die vorliegende Erfindung betrifft eine Zubereitung auf einer wässrigen Biopolymer- Chitosan/Eiweiß und vor allem Zuckertensid-haltigen Grundlage mit einer besonderen mizellenartigen Assoziationsstruktur zwischen Biopolymer (Chitosan/Gelatine) und Tensid, welche Schaum-Blasen mit hohem Gasaufnahmevermögen bilden, die zu stabilen Schäumen mit definierten physikalischen und morphologischen Eigenschaften verschäumbar sind. Solche Schäume sind für die Anwendung auf der Haut, in der Medizin, Life Science und in der Kosmetik verwendbar.

## Beschreibung

### Gegenstand der Erfindung

Die vorliegende Erfindung betrifft eine Zubereitung auf einer wässrigen Biopolymer- Chitosan/Eiweiß und vor allem Zuckertensid-haltigen Grundlage, welche zu stabilen und vorzugsweise sauerstoffhaltigen Schäumen mit definierten physikalischen und morphologischen Eigenschaften wie insbesondere Sauerstoffaufnahmekapazität, Schaumstabilität, verschäumbar sind. Solche Schäume sind für die Anwendung auf der Haut, in der Medizin, Life Science und in der Kosmetik verwendbar. Insbesondere kann die Zubereitung verschiedene Wirkstoffe (u.a. Arzneimittel) umfassen, welche erfindungsgemäß durch die Haut transportiert werden können. Die Zubereitung bzw. der vor allem O₂-Schaum ist insbesondere bei manifestierten hypoxischen (Sauerstoffmangel) Hautzuständen im kosmetischen Bereich, aber auch im therapeutischen Bereich, wie vor allem in der Wundbehandlung bei chronischen Wunden und Problemhaut, einsetzbar. Ein solcher kostengünstiger effizienter Einsatz im Rahmen der sog. Integralen Sauerstoff Therapie (IOT) ist sowohl ambulant als auch stationär möglich, was bei den bekannten aufwendigen Therapieverfahren z.B. Hyperbare Sauerstofftherapie (HBO) und Transkutane Sauerstofftherapie (TCOT) nicht möglich ist.

Die aufschäumbaren Zubereitungen bestehen neben Wasser aus einer Kombination wasserlöslicher Biopolymere, ausgewählt aus Chitosan oder Chitosansalz mit einer MM von vorzugsweise 70 kDa bis 2 000 kDa, sowie Eiweißen, ausgewählt aus Kollagen, Gelatine, oder Mischungen hiervon, zusammen mit einem oder mehreren nichtionischen Tensiden, insbesondere Alkyl-Glucosiden, sowie ggf. Wirkstoffen und Hilfsstoffen. Diese Zubereitungen können erfindungsgemäß mittels Versprüh-Vorrichtungen, vor allem mit einer Kombination aus einer Düse und einem Filter verschäumt und versprüht werden. Die Zubereitungen bilden aufgrund der spezifischen Inhaltsstoffe Produkte mit variabler einsatzflexibler Schaumzellengröße/Gas-Speicherzellenverteilung (Blasenverteilung), mit den damit verbundenen Anwendungseigenschaften. Es wird angenommen, dass aufgrund der besonderen Assoziationsstruktur zwischen Biopolymer (Chitosan/Gelatine) und Tensid mizellenartige Strukturen entstehen, welche dann Schaum-Blasen mit hohem Gasaufnahmevermögen (Gas/-Sauerstoffspeicherzellen), insbesondere Sauerstoff(O₂) bilden. Aus diesen kann bei topischer Applikation auf der Haut, vor allem Sauerstoff in ausreichender Menge in die Haut diffundieren unter Bildung eines bedarfsdefinierten O₂-Gradienten. Die Schäume ermöglichen erfindungsgemäß eine effiziente Hautpenetration von kosmetischen und pharmazeutischen Stoffen in ausreichender Menge für ihre erwarteten Wirkungen, oft potenziert durch eine synergetische Wirkung von Sauerstoff.

### Hintergrund der Erfindung

Hautbehandlungen mit unterschiedlichen Zielen und Wirkstoffen erfolgen üblicherweise topisch. Bei der topischen Versorgung mit Sauerstoff besteht einerseits das Problem, dass Sauerstoff wegen seiner geringen Löslichkeit zunächst nur als Gas zur Verfügung steht, andererseits aber auch toxisch wirken kann, wie z.B. dessen reaktive Formen (Radikale), die als Auslöser vieler Störungen oder Krankheiten (sog. oxidativer Stress), u.a. bei degenerativen Erkrankungen wie Arteriosklerose, Rheumatoide Arthritis, Diabetes mellitus, Colitis ulcerosa, vor allem auch bei chronischen Wunden oder auch bei Hautveränderungen und Problemhaut wie Cellulite, Altershaut, Bindegewebsveränderung gelten.

### Stand der Technik

Zur topischen Anwendung mit Wirkstoffen und Sauerstoff sind vor allem sauerstoffhaltige Perfluorcarbon- oder Hämoglobin-Lösungen (künstlichem Blut) bekannt, insbesondere bei der Wundbehandlung wie in EP 1696971 oder EP 1 513 492 beschrieben.

Daneben sind kosmetische Zubereitungen bekannt, welche Sauerstoff liposomal verkapselt enthalten, siehe WO 2005/ 016309 A1.

Des Weiteren kann Sauerstoffgas auch aus Siliciumperoxid oder H₂0₂-Katalyse generiert werden wie in US 6767342, US 8986725 bzw. US 20080274206 beschrieben.

Diese Verfahren sind jedoch oft aufwendig. Außerdem besteht das Problem, dass Haut eine schwer zu überwindende Barriere für die Diffusion von Stoffen, vor allem von hydrophobem Sauerstoffgas darstellt. Die Diffusionskonstante des Sauerstoffs in wässrigem Milieu der Haut ist so klein, dass bereits bei einem Diffusionsabstand von nur 20 µm die Diffusionskonstante nur 5% des anfänglichen Werts beträgt. Ändert sich die Haut durch Alter (Strukturverfestigung) oder Wunden (Flüssigkeitsfilm), so ist die Diffusionsbarriere noch höher.

Zur Überwindung der Hautbarriere wurden daher Emulsionssysteme entwickelt. Diese können auch z. T. aufgeschäumt werden, z. B. in Form von Aerosolschäumen, die eine halbfeste Formulierung umfassen, in einer Aerosoldose verpackt und durch ein Treibmittel unter Druck gesetzt werden. Kosmetisch eingesetzte Schäume beruhen auf einer Emulsionsbasis aus Wasser mit Ölen und ionischen, vor allem kationischen oder anionischen Tensiden. Derartige Tenside sind zwar schäumend, aber oft nicht hautverträglich.

Die US 20150374877 (EP 2 340 002) beschreibt schäumende Zubereitungen, bestehend aus Chitosan, einer Säure, einem Karbonatsalz und Tensiden wie kationisches Benzalkoniumchlorid, die durch Einblasen, Rühren, Mixen, oder Schütteln zur CO₂-Gas-Bildung führt. Dadurch bildet sich ein Schaum. Dieser wird jedoch nicht direkt angewendet, sondern zu einem Festschaum (Wundauflage/Scafold) gefriergetrocknet.

In der US 2015314037 (EP 2 928 511) werden feste hämostatische gefriergetrocknete Schäume mit Albumin beschrieben.

Andere Methoden zur Erzeugung von Schäumen umfassen besondere Vorrichtungen hierzu, wie die Injektion von Luft oder anderen Gasen über einen porösen Körper (Keramik- oder Glas-Membran) oder das Kondensationsverfahren (klassisches Aerosolverfahren) unter Druckveränderung. Dabei werden Flüssigkeiten unter Druck mit komprimiertem Gas gesättigt. Bei Druckabfall (mittels einer Düse) wird das gelöste Gas in Form von Blasen freigesetzt.

In der DE 10221449 (EP 1362576 A1) wird ein Aerosolschaum oder Pumpschaum zur Haarbehandlung beschrieben. Dabei handelt es sich um eine wässrige Zubereitung, umfassend eine Kombination aus einem kationischen Cellulose-Derivat und Chitosan, welche aus einem Aerosol-Druckbehälter mit oder ohne Treibmittel verabreicht wird. Hiermit soll das Problem der verbesserten Haarfestigkeit und Pflege-Eigenschaften bei insbesondere färbendem, jedoch nicht oxygenem, Schaumauftrag gelöst werden. Besondere Vorrichtungsmerkmale zur Verschäumung sind hier nicht beschrieben. Als Treibmittel (Treibgas) kommen hier bzw. üblicherweise wie z. B. auch in pharmazeutischen Aerosolen, Kohlenwasserstoffe, Fluorchlorkohlenwasserstoffe und komprimierte Gase (CO₂, NO und andere Gase) zum Einsatz.

In der EP 1 331 031 wird eine Vorrichtung zur Erzeugung von medizinischem Schaum aus einer Flüssigkeit, insbesondere einem Sklerotisierungsmittel und einem Gas, insbesondere Luft, beschrieben, welche eine mit der Schaumdüse verbundene Gas-Zuführeinrichtung und eine mit der Schaumdüse verbundene Flüssigkeitszuführeinrichtung aufweist, welche dadurch gekennzeichnet ist, dass in der Gas-Zuführeinrichtung eine Filtereinrichtung angeordnet ist. Damit sollen bestimmte Arzneimittel steril verabreicht werden. Der Stand der Technik zeigt, dass für die topische Versorgung von Hautarealen oder Problemhaut, insbesondere hypoxischer Haut, mit notwendigen Substraten und/oder Wirkstoffen und insbesondere Sauerstoff, wie dies im Falle eines kutanen hypoxischen Hautzustandes (tpO₂ weniger als 20 mm Hg) erforderlich wäre, ein Bedarf an Produkten besteht, welche sowohl hautverträglich als auch vorzugsweise wirksam im Zusammenhang mit der Versorgung mit Sauerstoff sowie leicht und sicher in der Anwendung sind. Insbesondere besteht Bedarf an einer einfachen effektiven Herstellung sowohl anwendungs- als auch einsatzflexibler Produkte.

### Aufgabe der Erfindung

Ausgehend von diesem Stand der Technik stellt sich die Aufgabe, eine topisch applizierbare, versprühbare, insbesondere verschäumbare Zubereitung zur Verfügung zu stellen, welche auf einfache Weise erhalten und angewendet werden kann, um Haut bzw. einem betreffenden Hautareal, vor allem unterversorgter, z. B. hypoxischer Haut oder Problemhaut, Altershaut (degenerierte Haut), vorzugsweise Wirkstoffe oder Arzneistoffe und vor allem Sauerstoff in ausreichender Menge zuzuführen. Dabei soll die Penetration von Wirkstoffen und auch Sauerstoff in die Haut unbedenklich (risikoarm) sein und vor allem auf Hautreizende Stoffe wie ionische Tenside, Silikonöle, Mineralöle, Fette verzichtet werden.

### Lösung der Aufgabe

Diese Aufgabe wird erfindungsgemäß gelöst durch eine versprüh- vor allem verschäumbare Zubereitung, bestehend aus einem wässrigen Träger, einer Kombination aus wasserlöslichen hautverträglichen, bioabbaubaren Naturpolymeren, nämlich Chitosan und Eiweißen, ausgewählt aus Kollagen, Gelatine oder Mischungen hiervon, insbesondere Gelatine, wie jeweils nachfolgend beschrieben, und einem oder mehreren nicht ionischen Tensiden, vor allem Alkyl-Glucosiden, polyoxylierten Fettsäuren oder derartigen Fettalkoholen (polyoxylierte Fettsäureester oder Fettsäurether = Macrogole). Dazu sind keine schäumenden ionischen (ionogenen) wie vor allem anionische, kationische oder quaternäre Tenside oder quaternäre Polymere wie Polyquat oder quaternisierte Celluloseverbindungen wie Polyquaternium Produkte u.a. nötig. Derartige kationische oder anionische Tenside und/oder quaternäre Polymere wie vor allem quaternäre Acrylate oder quaternisierte Celluloseverbindungen sind daher vorzugsweise in anmeldungsgemäßen Zubereitungen nicht vorhanden.

Überraschend wurde gefunden, dass die Kombination von Chitosan und Gelatine/Kollagen in bestimmten Verhältnissen zusammen mit nichtionischen Tensiden, die keine chemische Wechselwirkung mit Chitosan und Gelatine aufweisen, zur Bildung stabiler wässriger Blasensysteme wie Schäume unter hoher Gas- vor allem- Sauerstoffeinlagerung führt (Tabelle 3).

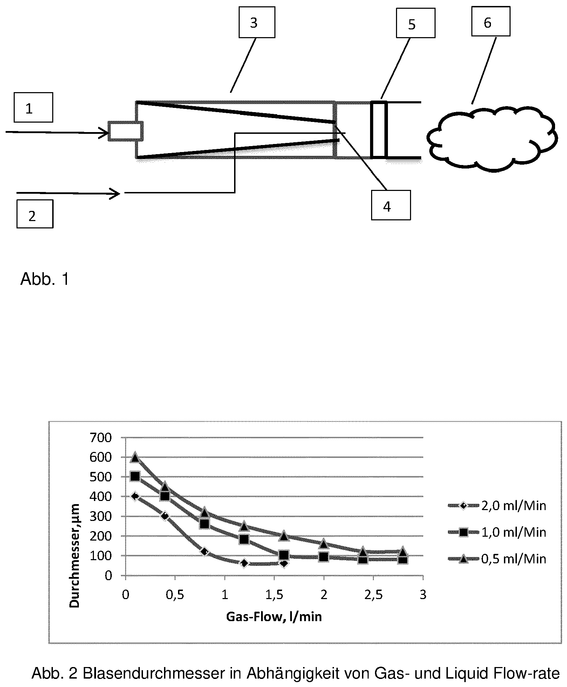

Die Zubereitung kann in einer geeigneten Applikations-Vorrichtung, z. B. mit einem Behälter mit Zuführleitung für die Zubereitung, Gas-Zuführleitung, Düse und Filterschaumkopf eingebracht und daraus verabreicht werden (Abb.1). Dazu kann als Treibgasquelle ein unter Druck stehendes Gas eingesetzt werden, das vorzugsweise zu mindestens 25 Vol. % aus Sauerstoff besteht. Die Zuführströme (Gas bzw. Zubereitung) stehen so angepasst miteinander in Strömungsverbindung, dass das Treibgas die versprühbare Zusammensetzung durch eine Düse optimal zerstäubt. Insbesondere kann eine Düse mit Schaumkopf mit porösem Filter eingerichtet werden, mittels der durch Gas/Liquid Filtration (also gleichzeitige Filtration von Zuführgas und aerosoliertem Flüssigkeitsstrom (Zubereitung)) aus der Vorrichtung ein bedarfsdefinierter Schaum herausgebildet wird.

Die Erfindung betrifft daher eine stabil versprüh- und verschäumbare Zubereitung, eine diese enthaltende Applikations-Vorrichtung und deren kosmetische und/oder therapeutische Anwendung zum stationären oder ambulanten Einsatz, insbesondere für Haut und Anhangsgebilde wie Nägel und Haare, z. B. wie Problemhaut, Wunden, entzündete Haut, Altershaut, Narbenhaut, degenerierte Haut bei Mensch und Tier, vorzugsweise zur Verabreichung von Wirkstoffen, und/oder Arzneistoffen, und/oder Substraten, und/oder Sauerstoff, vor allem Wirk-/Arzneistoffen in Verbindung mit Sauerstoff.

Andere Anwendungsbereiche bzw. Indikationen sind insbesondere Hauterkrankungen mit viralen, bakteriellen- und Pilzinfektionen, Psoriasis, atopische Dermatitis, Neurodermitis, Hauttumore, entzündliche Haut, Parodontitis, chronische Wunden bedingt durch Diabetes und chronisch-venöse Insuffizienz (CVI), entzündlich-rheumatische Erkrankungen (rheumatische Arthritis, chronische Polyarthritis) oder Schmerzlinderung/Analgesie. Insbesondere betrifft die Erfindung die topische Anwendung der Zubereitung zur kosmetischen oder pharmazeutischen Verabreichung von Wirkstoffen bei hypoxischer Problemhaut, vor allem bei chronischen Wunden (verschiedener Ätiologie) in allen Wundheilungsphasen, oder auch in Kombination mit anderen Verfahren wie die photodynamische Therapie (PDT) oder Sonophorese, was sowohl zur Wirkungsversteigerung als auch zur Kostenreduktion beitragen kann (im Rahmen eines sog. Integrated Oxygen Therapy (IOT)-Konzeptes).

Dabei hat sich gezeigt, dass das (kosmetische) Hauterscheinungsbild und/oder Heilungsprozesse u.a. die Geweberegeneration, insbesondere bei problematischer oder geschädigter Haut wie Akne, Altershaut, degenerierte Haut, Wunden und Narben, durch die erfindungsgemäße Zubereitung aufgrund geregelter Kollagensynthese und Angiogenese (Revaskularisation) erheblich verbessert werden kann. Dies steht im Gegensatz zur Lehre des Standes der Technik, wonach insbesondere Narben durch Abdeckung/Bandage, also durch Ausschluss von Luft/ Sauerstoff behandelt werden.

Die anmeldungsgemäßen Zubereitungen können sowohl in kosmetischen Betrieben wie in Krankenhäusern oder Arztpraxen angewendet werden. Aufgrund der Einsatzflexibilität und der Möglichkeit, mehrere Anwendungsschritte in einem System zu kombinieren, können Behandlungskosten wesentlich reduziert werden.

### Abbildungen

Abb. 1 zeigt eine Applikations-Vorrichtung für eine erfindungsgemäße Zubereitung;
Abb. 2 zeigt die Entwicklung von Schaumzellenblasen (hier Sauerstoff)- einer erfindungsgemäßen verschäumten Zubereitung im Zusammenhang mit Steuerparametern (Gas und Liquid Flow). (Beispiel 3);
Abb.3 zeigt die Stabilität des aus der Zubereitung generierten Schaumes über gewisse Zeiträume im Zusammenhang mit der Tensidkonzentration (Beispiel 6);
Abb. 4 zeigt die Gas(Sauerstoffgas)- Blasenverteilung in einer Zubereitung im Zusammenhang mit dem Chitosan-Gelatine-Polymergehalt (Beispiel 7), wobei sowohl breite als auch enge Blasenverteilungen erzielt werden können und die damit verbundene Schaumstabilität gezielt gesteuert werden kann;
Abb. 5 zeigt die Penetration von Sauerstoff in die Haut (tP O₂) mittels einer erfindungsgemäßen Zubereitung (Beispiel 8);
Bild. 6 zeigt die Behandlung von Onychomykose mit Curcumin-haltiger verschäumter Zubereitung gemäß Beispiel 12;
Abb. 7 zeigt den Verbesserungszustand der Haut nach Anwendung einer verschäumbaren Zubereitung bei Anwendung bei Psoriasis von 36 Patienten gemäß Beispiel 13;
Abb. 8 zeigt die kosmetische Wirkung von Minoxidilhaltiger verschäumter Zubereitung auf Haarwuchs gemäß Beispiel 14.
Abb. 9 zeigt die Struktur einer erfindungsgemäßen Zubereitung nach 12 Stunden Reifung gemäß Beispiel 1, Rez.1, verschäumt gemäß Beispiel 5 (Gas-Flow=0,7 I/min, Liquid-Flow=1,0 ml/min) (Maß Balke ist 1,5 mm. Blasendurchmesser ca. 2,0 mm).

### Nähere Erläuterung der Erfindung

Die erfindungsgemäße, einen Bio-Polymerschaum generierende versprühbare Zubereitung (Z) ist eine Chitosan/Eiweiß-Kombination mit integrierbaren Hilfs- und Wirkstoffen und besteht aus:
a) 0,1 bis 5,0 Gew. % Chitosan, vorzugsweise deacetyliertes Chitosan, MM 70.000 bis ca. 2.000.000 Da (70 KDa bis 2000 KDa), vorzugsweise mit einem Deacetylierungsgrad von 60-98%, insbesondere von mehr als 70, insbesondere mehr als 75%,
b) 0,1 bis 5,0 Gew.%, Eiweiß, ausgewählt aus Kollagen, vor allem Kollagen Typ I,II und III, Gelatine, Kollagenhydrolysaten oder Gelatine mit mittlerem MW von 20 kDa bis 60 kDa, oder Mischungen hiervon, insbesondere Gelatine der pharmazeutischen Qualität mit Bloom-Wert von 200 bis 300,
   wobei Chitosan und Eiweiß (Kollagen und/oder Gelatine) im Mengen-Verhältnis 4:1 bis 1:4 vorliegen;
c) 0,1 bis 5,0 Gew.% Säure, ausgewählt aus Mono- oder Bi- oder Trifunktionellen Carbonsäuren, welche eine oder mehrere Hydroxy-, Carboxy- Aminofunktionelle Gruppen oder Mischungen hiervon aufweisen, ausgewählt aus Essigsäure, Propionsäure, Milchsäure, Äpfelsäure, Bernsteinsäure, Malonsäure, Fumarsäure, Ascorbinsäure, Glutaminsäure, Salicylsäure, Pyrrolidoncarbonsäure oder Mischungen hiervon wie nachfolgend erläutert;
d) 0,1 bis 5,0 Gew. % nichtionische Tenside, ausgewählt aus C₆ -C₂₂ Alkylglucosiden, C₆ -C₂₂ -Alkylpolyglceriden, Polyglyceryl-C₆ -C₂₂- Fettsäureestern, polyoxylierten Polyglyceriden, polyoxylierten C₆ -C₂₂ -Fettsäuren, polyoxylierten C₆ -C₂₂ -Fettalkoholen, C₆-C₂₂-Fettalkoholpolyglykolethern, insbesondere ausgewählt aus C₆- C₂₂-Alkylglycosiden, C₆-C₂₂-Alkyl-polyglykosiden, Sacharose- C₆-C₂₂-Alkylestern, C₆-C₂₂- Fettalkoholpolyglykolethern oder Mischungen hiervon,
e) 0,01 bis 20 Gew. %, vor allem bis 10 Gew.% Hilfsstoffe ausgewählt aus Viskositätsregulatoren, Antioxidantien, Farbstoffen, Konservierungsmitteln, pH-Regulatoren, Lösungsvermittler oder Mischungen hiervon;
f) 0 bis 20 Gew.%, vor allem 0,01 bis 20 Gew. % Wirkstoffe, ausgewählt aus kosmetischen Wirkstoffen, pharmazeutischen Wirkstoffen [Arzneimitteln (API)] oder Mischungen hiervon;
g) 60 bis 95 Gew. % Wasser oder wässrige Lösung,
wobei die Zubereitung, insbesondere verschäumte, versprühte Zubereitung, Strukturen, vor allem mizellenartige Chitosan-Tensid-, Strukturen, mit einer homogen einstellbaren differenzierbaren Schaumzellengröße im Bereich von 0,01 bis 2,0 mm aufweist bzw. ausbildet.

Mit der erfindungsgemäßen aufschäumbaren stabilen Zubereitung bietet sich die Möglichkeit, wässrige strukturierte flüssige Blasensysteme wie Schäume (eine Kombination von einem in einer Polymermatrix stabilisierten Gas) zu erzeugen. Insbesondere weist die Zubereitung mizellenartige Chitosan-Tensid- Strukturen mit einem Gas-, vor allem Sauerstoffgas- Aufnahmevermögen von 10 bis 100 ml Gas, vor allem O₂ pro 1 bis 3 ml Zubereitung, auf.

So ist es möglich, morphologisch definierte Schäume u.a. mit definierten schmalen oder breiteren Blasen (Schaumzellendurchmesserverteilung) zu erzeugen, ohne dass dazu ein besonderer technischer oder apparativer Mehraufwand oder Umgestaltung nötig wäre. Insbesondere können homogene Schäume mit bis zu ca. 85 % aller Blasen (80-90 % Schaumzellen) mit ca. 0,1 bis 0,2 mm Mitteldurchmesser (z. B. 1,5% Chitosan in der Zubereitung), oder z.B. bis ca. 90 % Blasen (Schaumzellen) mit mittlerem Durchmesser von 0,3 bis 0,6 mm (1 % Chitosan in der Zubereitung) im Gegensatz zu Zubereitungen (z. B. 0,5 % Chitosan in der Zubereitung) mit sehr breiter Blasenverteilung mit mittlerem Durchmesser von 0,7 bis 1,2 mm hergestellt werden, je nach Anforderung oder Applikationsziel (enger Bereich, breite Fläche o.ä.). Ähnliches ergibt sich mit dem Einfluss der Tensidkonzentration in der Zubereitung.

Aufgrund des Einsatzes von natürlichen Polymeren in wässriger Lösung (Hydrogele von Biopolymeren) und besonders hautverträglichen Inhaltsstoffen können die Produkte in biomedizinischen und kosmetischen Anwendungen eingesetzt werden, ohne bisher bekannte nachteilige Effekte zu erzielen. Aufgrund der Zusammensetzung der Zubereitung können Schäume mit gewünschten und definierten Eigenschaften je nach Einsatzanforderung einfach und reproduzierbar erzeugt und so an den jeweils bestehenden Bedarf leicht angepasst werden. Dies ist z. B. besonders wichtig bei kosmetischen Anwendungen unterschiedlicher Zielrichtung wie Problemhaut oder degenerierter Haut. Der Einsatz in der Wundbehandlung bei allen Wundheilungsphasen ermöglicht eine effiziente und kostensparende Behandlung im Rahmen der sog. Integralen Sauerstoff-Therapie (IOT).

Die erfindungsgemäße Zubereitung auf Basis von Chitosan- und Eiweiß-vor allem Gelatine-Lösungen in den angegebenen Mischungsverhältnissen können aufgeschäumt und versprüht werden, z. B. mittels geeigneter Applikatoren wie Aerosol-Vorrichtungen, insbesondere Aerosol/Filtrations-Versprühvorrichtungen gemäß Abb.1.

Überraschend wurde auch gefunden, dass die erfindungsgemäße Zubereitung große Mengen von gasförmigen Sauerstoff aufnehmen und im generierten Schaum aufgelöst, also dispersiv verteilt enthalten kann, z. B. von 10 bis 100 ml O₂ pro 1 bis 3 ml Zubereitung, je nach Behandlungsziel und Behandlungsmodus.

Die aufschäumbaren bzw. aufgeschäumten Zubereitungen zeigen eine effiziente Penetration von Wirkstoffen in der Haut, in für die beabsichtigte Therapie ausreichender Menge.

Ausführliche Beschreibung der Erfindung und Inhaltsstoffe

### 1. Biopolymere

Als Biopolymere werden Chitosan und Abkömmlinge hiervon (z.B. Chitosan in eine SalzForm) sowie strukturelle Eiweiße (Proteine) eingesetzt.

Bevorzugte strukturelle Eiweiße (Proteine) sind vor allem Gelatine und Gelatinehydrolysat mit Pharmaqualität aus Schwein- oder Rinder Haut. Gelatine ist die denaturierte Form von fibrillärem Kollagen Typ I, II und/oder III. Kollagen vom Typ I, II, III kann ebenfalls eingesetzt werden.

Bevorzugt wird Chitosan (insbesondere kommerziell erhältliches mit Pharmaqualität) mit einem Molekulargewicht von etwa 70 kDa bis etwa 2 000 kDa verwendet. In einer weiteren bevorzugten Ausführungsform weist Chitosan ein Molekulargewicht zwischen etwa 100 kDa und etwa 1.000 kDa auf, vorzugsweise 400 kDa bis 900 kDa und insbesondere von 300 bis 800 kDa.

Bevorzugt wird ein deacetyliertes Chitosan wie beschrieben vor allem mit einem Deacetylierungsgrad von 60 bis 98%, insbesondere von 70 bis 98%, vor allem von 75 bis 98%. Ganz besonders bevorzugt wird Chitosan mit MM von 100 kD bis 500 kDa oder vor allem 100 kDa bis 600 KDa, und einem Deacethylierungsgrad (DA) von 80% bis 95%.

Als Protein (Eiweiß) wird vor allem Gelatine, z.B. Typ A und B mit einem Bloom-Wert von 200 bis 300, z.B. MedellaPro® (Gelita GmbH, Deutschland) verwendet. Alternativ oder zusätzlich kann auch Kollagen vom Typ I, II, III oder ein Gemisch hiervon eingesetzt werden.

Alternativ kann Chitosan teilweise, z. B. bis 45 Gew-%, bezogen auf die Menge an Chitosan ersetzt oder ergänzt sein durch andere Biopolymere, umfassend geeignete Glycosaminoglycane u.a. Hyaluronsäure, Chondroitin, Chondroitin-4-sulfat, Chondroitin-6-sulfat, Dermatansulfat, Keratansulfat, Heparinsulfat und Heparin, oder geeignete Mischungen hiervon, natürliche Polysaccharide aus der Gruppe, umfassend: Alginat, Dextran, Xanthan, Alginsäure, Agar (Galactose-Polymer), Carragenan, Carboxymethyl-, Isopropylcellulose, Cellulose, Pektin, Stärke oder geeignete Mischungen hiervon. Es kann Stärke in irgendeiner Form vorliegen, einschließlich α-Amylose und Amylopektin.

Alternativ oder gleichzeitig kann ein Teil (z. B. bis zu 45 Gew.%, bezogen auf die gesamte Eiweißmenge) des gewählten Eiweißes ersetzt oder ergänzt werden durch strukturelle Eiweiße der Gruppe aus Albumin, Lactoglobulin, Fibronektin und Mischungen hiervon. Dabei ist die Gesamtmenge an Polysaccharid bzw. Eiweiß wie oben in Anspruch 1 angegeben.

Vorzugsweise ist der Polysaccharid/Protein-, vor allem Chitosan bzw. Gelatine -Lösungsprozentsatz (Gew.% / Gew.%) in der wässrigen Ausgangszubereitung größer als 0,1% Chitosan bzw. Gelatine und weniger als 5,0% Chitosan bzw. Eiweiß wie Gelatine. Mehr bevorzugt ist eine Chitosan-Gelatine-Konzentration in der Lösung von mehr als je 0,3% und weniger als je 4,5%. Am meisten bevorzugt wird die Chitosan/Eiweiß, insbesondere Chitosan/Gelatine-Konzentration in der Lösung von mehr als je 0,5% Chitosan/Gelatine und weniger als je 4 % Chitosan / Gelatine.

Verhältnisse Chitosan/ Eiweiß, vor allem Gelatine (Gew./Gew.) sind insbesondere: 20/80 bis 80/20. Am meisten bevorzugt werden Chitosan/Eiweiß, vor allem Gelatine-Verhältnisse von 50/50 (1:1).

Chitosan kann industriell einfach aus Chitin von Crustacea, z.B. aus Krabben- oder Krillpanzern gewonnen werden. Es wird erfindungsgemäß nicht chemisch (kovalent) durch Derivatisierung zu beispielsweise Hydroxyethyl-, Hydroxypropyl- oder Hydroxybutylchitosan verändert. Es liegt in natürlicher Form als Ausgangsmaterial vor und kann vorzugsweise durch bekannte Methoden deacetyliert werden.

Ein bevorzugtes Chitosan oder Chitosansalz z.B. Chitosan-pyrrolidoncarboxylat, welches beispielsweise unter der Bezeichnung Kytamer® PC von der Firma Amerchol, USA, vertrieben wird. Geeignet ist auch Chitosan einer pharmazeutischen Qualität ("ChitoPharm" von Fa. Chitopharm, Norwegen). Das enthaltene Chitosan hat ein Molekulargewicht zwischen 200.000 bis 300.000 und ist zu bis 85% deacetyliert.

Gelatine oder Kollagen wird vor allem aus Schweine- bzw. Rinderhaut gewonnen.

### 2. Säure

Als Säuren eignen sich mono- oder bi- oder trifunktionelle Carbonsäuren, welche eine oder mehrere Hydroxy-, Carboxy- Aminofunktionelle Gruppen enthalten. Diese werden insbesondere ausgewählt aus Essigsäure, Milchsäure, Äpfelsäure, Bernsteinsäure, Malonsäure, Fumarsäure, Ascorbinsäure, Propionsäure, Glutaminsäure, Salicylsäure, Pyrrolidoncarbonsäure, vorzugsweise Essigsäure, Bernsteinsäure, Milchsäure, Ascorbinsäure, Pyrrolidoncarbonsäure, Salicylsäure oder der Kombination zweier oder mehrerer dieser Säuren.

Vorzugsweise werden Milch-, Ascorbin-, Pyrrolidoncarbon- oder Bernsteinsäure oder Mischungen hiervon ausgewählt in einer Menge von mehr als z. B. je 0,2% bis weniger als 4%. Stärker bevorzugt wird Bernsteinsäure und Milchsäure in einer Menge, entsprechend einem Säurelösungsprozentsatz (Gew./Gew., bezogen auf die Gesamtlösung) von mehr als 0,5% (Gew./Gew.) und weniger als 2,5%, jeweils.

### 3. Tensid

Als Tenside eignen sich vor allem nicht ionische Tenside, ausgewählt aus der Gruppe, umfassend ethoxylierte und/oder propoxylierte Fettalkohol-Ether (Fettalkoholpolyglycolether), ethoxylierte und/oder propoxylierte Fettsäure-Ester, ethoxylierte Triglyceride, bzw. hydrogenisierte Triglyceride oder insbesondere Zuckertenside wie Alkyl-Saccharoseester oder Alkyglucoside oder Alkypoly(glucoside), letztere mit 2-(Glucose-Einheiten), wobei in diesen Tensiden der Alkylteil des Esters bzw. Ethers von einer Fettsäure oder einem Fettalkohol abgeleitet ist und eine Länge von 6 bis 24, insbesondere 6-22 C-Atomen aufweisen kann. Dabei kann der Alkylteil gesättigt, einfach ungesättigt oder mehrfach ungesättigt sein wie Laurylsäure, Ölsäure, Linolsäure, Linolensäure, Myristinsäure. In den ethoxylierten und/oder propoxylierten Produkten kann der Ethoxylierungs- (EO-) bzw. Propoxylierungs- (PO)- Anteil 2 bis 24 %, insbesondere 7-16% betragen.

Besonders bevorzugte Tenside sind ausgewählt aus C₆-C₂₂- Alkylglycosiden, vor allem C₁₀-C₂₂- Alkylglycosiden, C₆-C₂₂- Alkylpolyglykosiden, Sacharose- C₆ -C₂₂-Alkylestern, C₆-C₂₂- Fettalkoholpolyglykolethern oder Mischungen hiervon. Derartige Tenside sind im Handel erhältlich. Auch pflanzliche Saponine bzw. Terpene (Seifenbaum, Seifenkraut, Licorize) eignen sich.

Insbesondere bevorzugt sind C₆-bis C₁₈- oder C₁₀-C₁₈-Alkylglucoside.

Vor allem geeignet sind die folgenden Alkyl-Glucoside oder Mischungen hiervon: CAPRYLYL/CAPRYL GLUCOSIDE mit einem Alkylteil aus einer (aus Kokosöl extrahierten) sehr kurzen Fettsäure mit 6-8 Kohlenstoffatomen, im Mittel C7.

DECYL GLUCOSIDE mit (aus Kokosöl extrahiertem) Alkylteil aus Caprinsäure mit 10 Kohlenstoffatomen.

LAURYL GLUCOSIDE mit (aus Kokosöl extrahiertem) Alkylteil aus Laurylsäure mit 12 Kohlenstoffatomen. Ein Handelsprodukt hiervon ist unter dem Namen Plantacare® bekannt.

COCO-GLUCOSIDE mit Alkylteilen aller Fettsäuren des Kokosöls (hauptsächlich Caprinsäure und Laurylsäure, sowie Ölsäure, Linolsäure, Linolensäure).

Vorzugsweise werden das oder die Tenside in der Zubereitung in einer Menge / Konzentration im Bereich von 0,1 % bis 4 Gew. % (W/W) eingesetzt.

Vorzugsweise beträgt das Verhältnis Tensid zu Chitosan oder Tensid zu Chitosan+Eiweiß 1 zu 10 bis 1 zu 3.

### 4. Wirkstoffe (pharmazeutische Wirkstoffe= API, kosmetische Wirkstoffe)

Erfindungsgemäß können sowohl hydrophile als auch lipophile dermatopharmazeutische Wirkstoffe eingesetzt werden. Bevorzugt weist eine Zubereitung 0 bis 20 Gew.%, insbesondere 0,01 bis 10 Gew.% eines oder mehrerer wasserlöslicher und/oder fettlöslicher Wirkstoffe auf, ausgewählt
- aus der folgenden Gruppe: Analgetika, Lokalanästhetika, Antiphlogistika (Antirheumatika), Glukokortikoide, Antibiotika, Antimykotika, Virustatika, Immunosupressiva, Immunomodulatoren, Antipsoriatika, Keratolytika, Hormone, Phytopharmaka (pflanzliche Extrakte, Phenolcarbonsäure, Cumarine, Lignane, Flavonoide, Isoflavone, Xanthophylle, Gerbstoffe, Anthranoide , Polyphenole, Alkaloide, Glykoside, Mono- und Sesquiterpene, Triterpene, Sterole, Carotinoide, Terpenoide, Phenolsäurederivate, ätherische Öle), Vitamine und Provitamine, Antioxidantien.
Insbesondere geeignet sind Wirkstoffe aus der folgende Liste:
- Antientzündliche Mittel (NSAID), wie Salicylate v.a. Alkyl- oder Benzylsalicylate, Indole, -Propionate, Pyrazole (Nifluminsäure ), Fenamate, Anthraniline;
- Analgetika, wie Opiate, Salicylsäure und Derivate, Gentisinsäure, Anthranilsäure und Derivate, Mefenaminsäure, Flufenaminsäure, Nicotinsäure und Derivate
- Analgetika/Antiphlogistika, insbesondere:
   -Antirheumatika wie Heretoaryl- sowie Arylessig- und Propionsäuren-Derivate (Indometacin, Diclofenac, Sulindac, Tolmetin, Ibuprofen, Ketoprofen, Flurbiprofen u.a.);
- Antifibrolytika wie ε-Capronsäure, Tranexamsäure, para-Aminomethylbenzolsäure u.a., Lidocain, Procain;
- Hormone wie Cortison und Derivate, Testosteron und Derivate, Östradiol und Derivate , Melatonin, Dehydroepiandrosteron;
- Aminosäuren und deren Salze: Cystein, Arginin, Creatin, Glutathion, Hydroxyprolin, Taurin; Citrullin, Ornithin, γ-Aminobuttersäure, Thyroxin, Homocystein, Adenosylmethionin, u.a.;
- Peptide (di-, tri-, tetra-, poly-): z. B. Karnitin, Glutathion, Ephitalamin u. a.;
- Vitamine A-, B-, E- und C-Gruppe und Vitamin-ähnliche Stoffe: Thiamin, Riboflavin, Niazin, Pantothensäure, Pyrridoxin, Folsäure, Kobalamin, Orotsäure, para-Aminobenzoesäure, Inosit, Bioflavonoide, Ascorbinsäure, Biotin, Alfa-Tocopherol, Retinol und Derivate, Beta-Karotin, Liponsäure;
- Antioxidantien (natürlichen/pflanzlichen- und synthetischen Ursprungs): Tocopherol, Carotin, Vitamin A oder Butylhydroxyanisol, Gallussäureester;
- Antivirale, fungizide und antibakterielle Mittel wie Aztreonam, Chlorhexidin-Gluconat, Amikacin, Daptomycin, Vancomycin;
- Wachstumsfaktoren oder andere biologische Stoffe, die die Wundheilung fördern.

Der oder die Wirkstoffe können bevorzugt eingesetzt werden in einer Menge von mindestens etwa 1 µg 25 µg 50 µg 100 µg 250 µg 500 µg 750 µg 1 mg, 5 mg, 10 mg, 25 mg, 50 mg, 75 mg, 100 mg. Z.B. 200 mg, 250 mg, 300 mg, 400 mg oder 500 mg, wie z. B. Antibiotika.

Als pharmazeutisch-therapeutische Wirkstoffe kommen insbesondere infrage: Diclofenac, Minoxidil, Clobetasol, Methotrexat, Propolis, Curcumin und andere Singlettsauerstoff generierende Farbstoffe (Methylenblau, Rose Bengal oder Riboflavin).

Als kosmetische Wirkstoffe können vor allem Hyaluronsäure mit verschiedenen Molekulargewichten von 10 kDa bis 1000 kDa, Chondroitinsulfat mit MW von 10 kDa bis 50 kDa, Vitamine, wie Vitamin E oder andere wie oben genannt, pflanzliche Extrakte (Aloe Vera, Centella asiatica und viele andere), ätherische Öle wie Lavendelöl, Rosmarinöl, Citrusöl, Orangenöl, Minzöl, Ylang Ylang, Jasminöl, und viele andere, bekannte natürliche Duftstoffe eingesetzt werden.

Besonders bevorzugt werden hier als Wirkstoffe Biopolymere bzw. Glucosaminoglucane als Bestandteile der extrazellulären Matrix (ECM) wie vor allem Hyaluronsäure von 10 kDa bis 500 kDa, Chondroitinsulfate als auch natürliche Polysaccharide bzw. Alginate verschiedenen MW, Xanthan, Pektin, Zellulosederivate sowie Hydroxyethyl- und Hydroxypropylzellulose oder Natriumcellulosemonoglycolat. Proteine wie Kollagen und Kollagenhydrolysate und auch Globuline (Albumin, Lactoglobulin).

Insbesondere bevorzugte Wirkstoffe oder Gerüststoffe sind Hyaluronsäure mit MW von 10 kDa bis 500 kDa Chondroitinsulfat, Alginsäure und Alginate.

### 5. Hilfsstoffe

Als Hilfsstoffe gelten It. Pharmakopöe ein Stoff oder Stoffgemische, der zur Herstellung von Arzneimitteln oder zur Unterstützung oder Regelung der Arzneimittelwirkung dienen und dem in der im Endprodukt enthaltenen Menge keine unmittelbare pharmakologische Wirkung zukommt.

Zu erfindungsgemäß verwendbaren Hilfsstoffen gehören u.a.:
Antioxidantien, Farbstoffe (natürliche und synthetische), Konservierungsmittel (z.B. Sensiva SC 50, Phenoxyathanol), pH-Regulatoren, Stoffe zur Beeinflussung der Viskosität und Konsistenz (Viskositätsregulatoren), Lösungsvermittler wie vor allem Ethanol, Isopropanol, Propylenglycol, Glycerin oder N-Methyl-pyrrolidon, in einer verträglichen Menge 0,1 von 20 %.

Erfindungsgemäß umfasst die Zubereitung vorzugsweise keine lipidischen Stoffe. Hieruntern werden Mono-, Di- Triglyceride von C₁₂₋₂₂ Fettsäuren oder auch natürliche Öle z. B. C₁₆₋C₂₂- gesättigte, oder ungesättigte Fettsäuren wie Olivenöl, oder Phospholipide verstanden.

Ätherische Öle (im allgemeinen Terpene, Terpenoide, Aromaten, die keine Fette (Lipide) enthalten) sind erfindungsgemäß keine lipidischen Stoffe und können als Wirkstoffe enthalten sein.

### 6. Treibmittel (Gas)

Zur Versprühung bzw. Aufschäumung der Zubereitung in geeigneten Vorrichtungen - wie beschrieben- werden Treibgase eingesetzt. Hierbei handelt es sich erfindungsgemäß vor allem um Sauerstoff oder ein sauerstoffhaltiges, insbesondere Sauerstoffreiches Gasgemisch mit einem Sauerstoffgehalt von vorzugsweise 25 Vol.-%, bevorzugt mindestens 60 Vol.-%und höher. Zumeist handelt es sich um weitgehend reinen Sauerstoff mit einem Gehalt von mindestens 98 Vol.-%.

Das Treibgas kann aber neben Sauerstoff auch andere Gase wie solche, die in Luft enthalten sind (Stickstoff, CO₂) enthalten bzw. dem Treibgas können andere Gase hinzugefügt werden. Es kann sich hierbei um kleinere Gas-Zugaben handeln, ebenso gut möglich ist jedoch die Verwendung von Gasen, die eine zusätzliche Wirkung entfalten können. Ein Beispiel für eine Zumischung eines Gases ist die Verwendung von Distickstoffmonoxid N₂O oder CO₂. Auch Inertgas wie Argon oder Xenon sind möglich.

Der vom Treibgas erzeugte Überdruck liegt typischerweise in einem Bereich zwischen 0,3 und 4 bar, vorzugsweise zwischen 0,5 und 3 bar, besonders bevorzugt bei 0,5 bis 2 bar. Auf diese Weise kann eine ausreichend starke Sauerstoffanreicherung in der Zusammensetzung und nachfolgend in der Haut, bzw. Dermis herbeigeführt werden.

Als Treibgasquelle eignen sich unterschiedliche Vorrichtungen, beispielsweise eine Sauerstoffflasche oder ein Sauerstoffgenerator. Das Applikationssystem kann auch an eine kontinuierlich Sauerstoff fördernde Leitung angeschlossen werden, so dass das System nahezu ununterbrochen betrieben werden kann.

Andere Treibgase wie sonst eingesetzt wie Butan, Pentan, andere Alkane, reines CO₂ sowie Gemische der vorstehend genannte Treibmittel sind nicht geeignet und daher erfindungsgemäß nicht verwendet.

### Herstellung der Zubereitungen

Die erfindungsgemäßen verschäumbaren Zubereitungen werden hergestellt durch einfaches Mischen der Ausgangsmaterialien vorzugsweise wie in den nachfolgenden Beispielen 1 bis 2 beschrieben. Dabei kann destilliertes Wasser erwärmt werden auf z.B. mehr als 40° C bis z.B. auf 70° C. Unter Rühren werden sodann das oder die Eiweiße /Proteine (z. B. Gelatine) zugegeben und bis vollständigen Auflösung gerührt. Die erhaltene klare Lösung wird abgekühlt bis auf 30° bis 40°C. Dieser Lösung wird unter Rühren die Säure(n) und danach das oder die Polysaccharide in der jeweils angegebenen Menge/Konzentration zugegeben. Die resultierende Mischung wird bis zur vollständigen Auflösung des Polysaccharides gerührt und bis auf Raumtemperatur abgekühlt. Die Lösung weist einen pH Wert < 7, insbesondere von 3 bis 6,8 auf.

Zu dieser Lösung wird sodann die vorgesehene Menge an Tensid wie vor allem Decylglucoside (z. B. 50%-Plantacare® 2000UP in Wasser, BASF) bei Raumtemperatur zugegeben und bis zur vollständigen Auflösung gerührt. Es bildet sich eine klare schaumfähige Zubereitung, insbesondere in Form einer Lösung.

### Applikations-Vorrichtung

Die erfindungsgemäße Zubereitung wird angewendet mittels einer geeigneten Applikationsvorrichtung, umfassend ein Applikationssystem (3) mit einem Reservoir-Behälter für die Zubereitung (Z), einer Zuführleitung (2) für die Zubereitung (Z), einer Treibgasquelle mit Zuführleitung (1) für Treibgas, eine Düse (4), wobei die Düse (4) vorzugsweise einen Schaumkopf (5) mit integriertem porösen Körper aufweist. Das Treibgas weist vor allem Sauerstoff oder mehr als 25 Vol.% Sauerstoff angereicherte Luft auf. Der Vorratsbehälter kann typischerweise zwischen 0,5 und 100 ml an versprühbarer Zubereitung (Z) enthalten.

Insbesondere wird die Zubereitung in einer Vorrichtung mit Venturi-Düse, aerosoliert. Es ist dabei von Vorteil, wenn die aerolisierte Zubereitung durch einen Filter versprüht wird. (Besondere bevorzugte Kombination von Aerosol-Verfahren und Gas/Liquid-Filtration). Mit der erfindungsgemäßen Zubereitung können somit Schäume mit bedarfsdefinierten physikalischen und morphologischen Eigenschaften hergestellt werden.

Venturi-Düsen sind an sich bekannt, jedoch bisher noch nicht in der Aeroliserung zusammen mit einer Filtration über einen Filterkopf zum Verschäumen einer Zubereitung eingesetzt worden.

In Abb.1. ist eine bevorzugte Applikations-Vorrichtung zum Verschäumen einer anmeldungsgemäßen Zubereitung (Z) beispielhaft dargestellt. Es bedeuten hier 1: Gasquelle mit Zuführleitung und Steuerung, 2: Zuführleitung und Steuerung der Vorratszubereitung (Z), 3: Ein Halter oder Applikator (3) für die Düse (4) und für die Leitungen von Gas (1) und flüssiger Zubereitung (2), 4: Venturi-Düse, 5: Schaumkopf mit Filtermaterial, 6: aus der Zubereitung (Z) gebildeter Schaum.

Die Applikations-Vorrichtung verfügt vor allem über eine Zweistoff-Venturidüse (4) zum Versprühen der Zubereitung (Z) mit Zuführleitung (2) sowie eine Treibgasquelle/ Treibgas-Zuführleitung (1), hier mit einer Treibgas- oder Sauerstoffzuführung über eine Leitung von extern. Das Treibgas treibt bei Betätigung des Applikationssystems die versprühbare Zubereitung durch die Sprühvorrichtung, vor allem Düse (4) und dadurch bildet sich ein Aerosol mit definierter Partikelgröße. Dabei bildet sich ein Sprühkegel aus feinen Aerosoltropfen. Bei der Düse handelt es sich vor allem um eine Zweistoff-Venturi- (= Lavall-) Düse. Während des nachfolgenden Fluid(=Liquid)/Gas-Filtrationsprozesses durch einen porösen Körper(5) bildet sich ein Schaum (6).

Der Schaumkopf (5) enthält vorzugsweise einen porösen, bzw. aus porösem Material bestehenden Filter mit bestimmter Porengröße, z. B. Edelstahl-Siebgewebe -50 µm (SFT GmbH, Deutschland), oder eine Fritte aus Polyethylen (HDPE, Porendurchmesser ca. 20 µm (Carl Roth GmbH, DE), oder Vlies (aus Viskose, Polyester oder Gemische mit Porendurchmesser ca. 10-25 µm (POLO Filtertechnik, DE), oder Filterpapier (aus Zellulose, Porendurchmesser ca. 1-5 µm (Carl Roth GmbH, DE). Zerstäubungsparameter (Druck und Flow) und Filtermaterial können jeweils ausgewählt werden. Geeignete poröse Materialien für Gas/Liquid Filter sind: Glas- und Keramikfilter, Sintermaterialien, Gewebe (z.B. Metall- oder Kunststoffgewebe), nicht gewebte Materialien (z.B. Flies). Vor allem die Anwendung von nicht gewebten Materialien (Vliese) aus Polyester/Viskose und anderen Polymeren ermöglicht die Generation von geeigneten Schäumen, wie in Tab. 3, Beispiel 3 erläutert.

Wie weiterhin ersichtlich, kann die erfindungsgemäße Zubereitung insbesondere auch ohne Anwesenheit kationischer und/oder anionischer Tenside zu einem geeigneten stabilen Schaum umgewandelt werden. Dabei können durch geeignete Auswahl der Düsenparameter (in erster Linie Bohrungsdurchmesser, Druck, Gas- und Liquid-Flow und Strahlform) sowie der Filtereigenschaften (Filterfläche, Porengröße, Porosität) aus den erfindungsgemäßen Zubereitungen stabile Schäume mit den gewünschten morphologischen/ physikalischen Eigenschaften erhalten werden.

Die Vorrichtung kann zwischen 0,5 und 100 ml aufzuschäumende Zubereitung im dafür vorgesehenen Behälter aufweisen.

Bei Aufschäumung der Zubereitung kann der Volumenfluss (je nach Druck 0,1 bis 50 ml/min) bevorzugt 0,3 bis 10 ml/min und besonders bevorzugt 0,5 bis 8 ml/min betragen. Typischerweise werden auf 100 cm² Hautoberfläche von 1 bis zu 4 ml der Zubereitung verschäumt und gesprüht.

Besonders bevorzugt ist es, wenn das Treibgas und die Zubereitung Z (flüssig, bis gelförmig) mit einem Druck von 0,1 bis 3,0 bar und mit einer Flow-rate 0,1 bis 40 I/Min verströmt werden. Ganz besonders bevorzugt ist ein Verhältnis von Gasstrom zu Flüssigkeitsstrom (Gas-Liquid-Flow)-Verhältnis von 10 zu 1 bis 100 zu 1.

Der erfindungsgemäße Schaum gewährleistet eine gute Penetration von Wirkstoffen und Substraten, vor allem auch O₂ in die Haut. Die vorzugsweise vorhandenen Wirkstoffe (kosmetisch oder pharmazeutisch-therapeutisch) können erleichtert penetrieren und ihre Wirksamkeit entfalten.

Vorzugsweise kann nach der Besprühung mit Schaum noch eine Begasung der Haut mit Sauerstoff angereichertem Treibgas (mindestens 25 Vol.% O₂) einige Zeit durchgeführt werden.

### Anwendungen

Die anmeldungsgemäßen Zubereitungen werden insbesondere als Schaum topisch eingesetzt. Dabei kann Haut in kleineren oder größeren Bereichen im Zusammenhang mit einer Degeneration, Schädigung durch Licht, Alter, Verletzung oder Krankheit kosmetisch oder therapeutisch behandelt werden. Zu den weitverbreiteten Hautkrankheiten gehören: Dermatomykosen, Nagelentzündung, Nagelpilz, Rheumatische Hauterkrankungen (psoriatische Arthritis), Hauterkrankungen durch Viren (Herpes, Zoster, Pocken, Papillom, Warzen, u.a.), Ekzeme (akute-, allergische, chronische-), Neurodermitis; weiterhin auch Geschwülste (Tumoren) der Haut wie Aktinische Keratose, Plattenepithelkarzinom und systemische Sclerosis (Sklerodermie).

Weitere Hauterkrankungen oder problematische Hautveränderungen sind Narbenbildung bzw. die Behandlung hiervon wie von atrophen und hypertrophen Narben, Keloiden und Falten.

Vorzugsweise werden die erfindungsgemäßen Zubereitungen verwendet bzw. eingesetzt bei Dermatomykosen, Nagelentzündung, Nagelpilz, rheumatischen Haut-Erkrankungen (psoriatische Arthritis), Hauterkrankungen durch Viren (Herpes, Zoster, Pocken, Papillom; Warzen, u.a.), Ekzeme (akute-, allergische, chronische-, Neurodermitis), Geschwülste (Tumoren) der Haut, insbesondere Aktinische Keratose, Plattenepithelkarzinom.

Je nach Indikation kann ein pharmazeutischer oder ein kosmetischer Wirkstoff eingesetzt werden oder Kombinationen hiervon.

Ein pharmazeutischer Wirkstoff/Medikament ist ein Stoff oder Stoffgemisch, dem die Wirkung eines verwendungsfertigen Arzneimittels zugesprochen wird und als solches eingesetzt wird. Ein kosmetischer Wirkstoff ist ein solcher, der die Haut schützt, pflegt und ernährt.

Eine kosmetische Anwendung von vor allem O₂-haltigen Schäumen kommt insbesondere infrage bei Narbenhaut, Altershaut (Falten), trockener Haut, empfindlicher, fleckiger Haut, auch zur Hautverfeinerung, Beruhigung, oder bei Rosacea, Vitiligo, Cellulite, Haarbehandlung wie Alopezia, Pigmentstörung, Nagelbehandlung oder Kombinationen hiervon.

Zur Hautpflege, u.a. bei kosmetischen Indikationen können beispielsweise auch Wirkstoffe wie Hyaluronsäure, oder auch Vitamine (A;C;E; B), Antioxidantien, Aminosäuren, Peelingmittel, Depilatoren, depigmentierende Witrkstoffe, Moisturizer, Haarwuchsmittel, Anticellulite-Mittel und viele andere kosmetische Stoffe u.a. pflanzlichen Ursprungs wie vorallem Aloe Vera, Centella asiatica und viele andere pflanzliche Extrakte und ätherische Öle wie Lavendelöl, Rosmarinöl, Citrusöl, Orangenöl, Minzöl, Ylang Ylang, Jasminöl, und viele andere bekannte natürliche Duftstoffe, Proteinhydrolysate und Peptide Carnitin, Glutathion, Ephitalamin u.a einzeln oder als Kombinationen mehrerer dieser Wirkstoffe eingesetzt werden.

Die erfindungsgemäßen Zubereitungen können wie beschrieben auf einfache Weise angewendet/aufgeschäumt und auf das zu behandelnde Areal mit einer geeigneten Applikations-Vorrichtung zum Verschäumen/Versprühen (Abb.1) aufgetragen werden. Insbesondere geeignet sind die Zubereitungen zur Applikation sowohl therapeutisch als auch kosmetisch auf Haut/Schleimhaut oder Problemhaut wie Akne, Narben, Falten, Wunden, chronische und infizierte Wunden, Verbrennungen, letzteres insbesondere auch ohne Wirkstoffe aufgrund der besonderen Beschaffenheit und Bioverträglichkeit der erfindungsgemäßen Zubereitung, wie im nachfolgenden Beispielteil näher ausgeführt.

### Vorteile der erfindungsgemäßen Zubereitung

Die erfindungsgemäße Zubereitung kann auf einfache Weise hergestellt und angewendet werden. Mit der erfindungsgemäßen Zubereitung können dabei Schäume reproduzierbar mit definierten Eigenschaften gebildet werden.

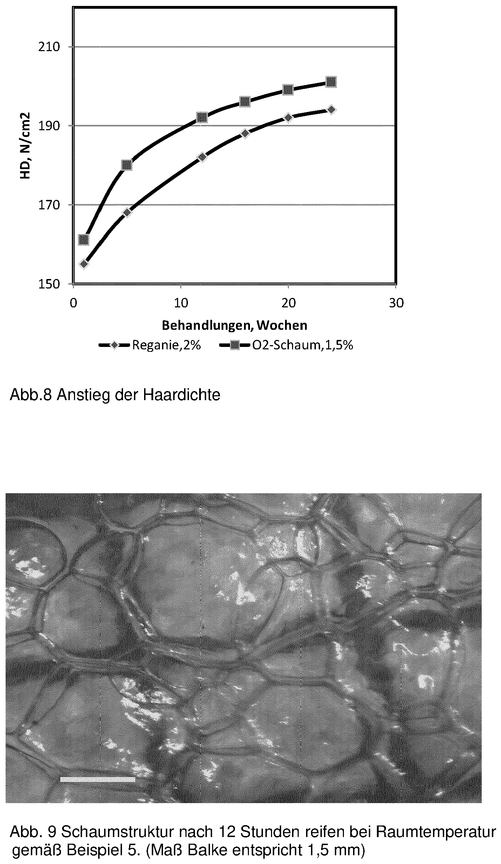

Durch Versprühen mit einer Applikations-Vorrichtung, vorzugsweise mittels Kombination von Aerosol-Verfahren und Gas/Liquid-Filtration, direkt auf die Haut/Anhangsgebilde wird gleichzeitig im Schaum dispersiv eingelagerter Sauerstoff appliziert, der zu einer Wirkungsverstärkung führen kann, da die O₂-Diffusion aus der Zubereitung erleichtert ist. Es wird angenommen, dass aufgrund Kombination aus Biopolymer, vor allem Chitosan, plus Tensid eine stabile mizellare Struktur vorliegt (erkennbar z.B. mittels Leitfähigkeitsmessung, vgl. Foto-Abb. 9, Blasenwandstrukturen), worin Gas, vor allem Sauerstoff, während der Schaumgenerierung eingelagert und nach Hautapplikation leichter herausdiffundieren kann. Andere vorstellbare Mechanismen für erleichterte Diffusion basieren auf der Laplasischen Gleichung. Durch Zerplatzen der Schaumzelle entsteht ein zusätzlicher Druck, der die Diffusion von Gas erleichtern kann.

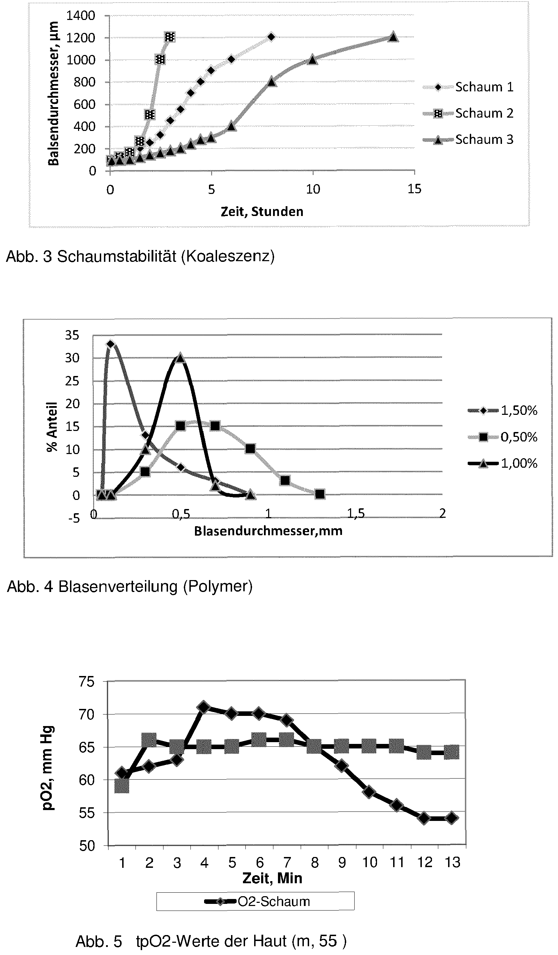

Die Zubereitungen sind bioverträglich und daher für breite Anwendungen in der Medizin und Life Science geeignet. Sie verfügen über eine gute Adhäsion an die Haut und Schleimhaut (Mucoadhäsion), (siehe Tab.5, Beispiel 9) und können auch in die Haut penetrieren, so dass damit Medikamente (APl) und auch kosmetische Wirkstoffe (für Menschen und Tiere), sowohl therapeutisch als auch kosmetisch für eine mucosale-, intrakutane- oder transkutane Gabe in ausreichender Menge verabreicht werden können, siehe Abb.5, Beispiel 8 und Tabelle 6, Beispiel 10.

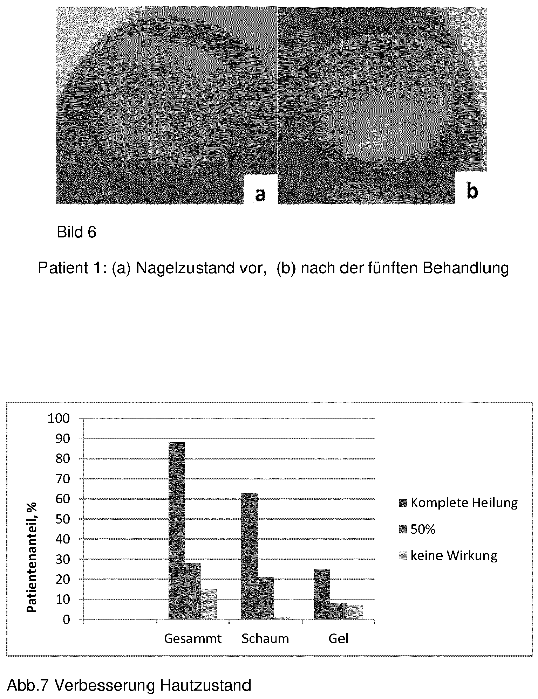

Ein weiterer überraschender Effekt ist, dass die erfindungsgemäßen Zubereitungen in Form des applizierbaren Sauerstoffschaumes auch ohne Wirkstoffe bei Hautkrankheiten (Beispiele: Kelloide, Wundheilung, Verbrennungen) ihre therapeutischen Wirkungen zeigen und dadurch auch die effiziente Heilung von chronischen und Brandwunden oder auch die Behandlung von Narben gewährleisten (Abb.6, Tabelle 6 und 7).

### Beispiele

Die Erfindung wird anhand der nachfolgenden Beispiele 1 bis 14 näher erläutert. Dabei betreffen die Beispiele 1 bis 7 die Zubereitungen und deren Schaumeigenschaften, und die Beispiele 8-14 Anwendungen.

### Beispiel 1. Zubereitungen 1-12 mit unterschiedlichen Tensiden

Die Rezeptur-Zubereitungen 1 bis 12 werden im gleichen Modus zubereitet: Destilliertes Wasser (981,0 g) wurde in einem 1,5 I Glasgefäß unter Rühren (Magnetrührer) auf 70°C erwärmt. Danach wurden 7,5 g Gelatine (Typ MedellaPro®, Gelita) zugegeben und bis zur vollständigen Auflösung gerührt. Die resultierende klare Lösung wurde bis auf 40°C abgekühlt. Zu dieser Lösung wurden unter mäßigem Rühren 4,0 g Michsäure (90%-ig) zugegeben und danach 7,5 g Chitosan-Pulver (Chitopharm). Die resultierende Lösung wurde bis zur vollständigen Auflösung von Chitosan gerührt und auf Raumtemperatur abgekühlt. Zu dieser Lösung wurden 2,0 g Decylglucoside (50%-Plantacare 2001 in Wasser, BASF) zugegeben und unter langsamem Rühren bis zur vollständigen Auflösung gerührt. Es bildete sich eine klare schaumfähige Lösung. Die resultierende Lösung hatte einen pH-Wert von ca. 5,5. Andere Tenside wurden in der Formulierung in ähnlicher Weise bei Raumtemperatur zugegeben.

Das eingesetzte Chitosan hatte eine mittlere molare Masse von ca. 810 KDa und eine Brookfield Viscosity von 286 cps. Die Gelatine hatte einen Gel Strength-Wert von 200-300 Bloom (Gelita GmbH).

Die Zusammensetzung der Zubereitungen und deren Schaumbildungsvermögen (wie nachfolgend erläutert) sind in Tabelle 1 (Ingredientien in %) zusammengestellt:

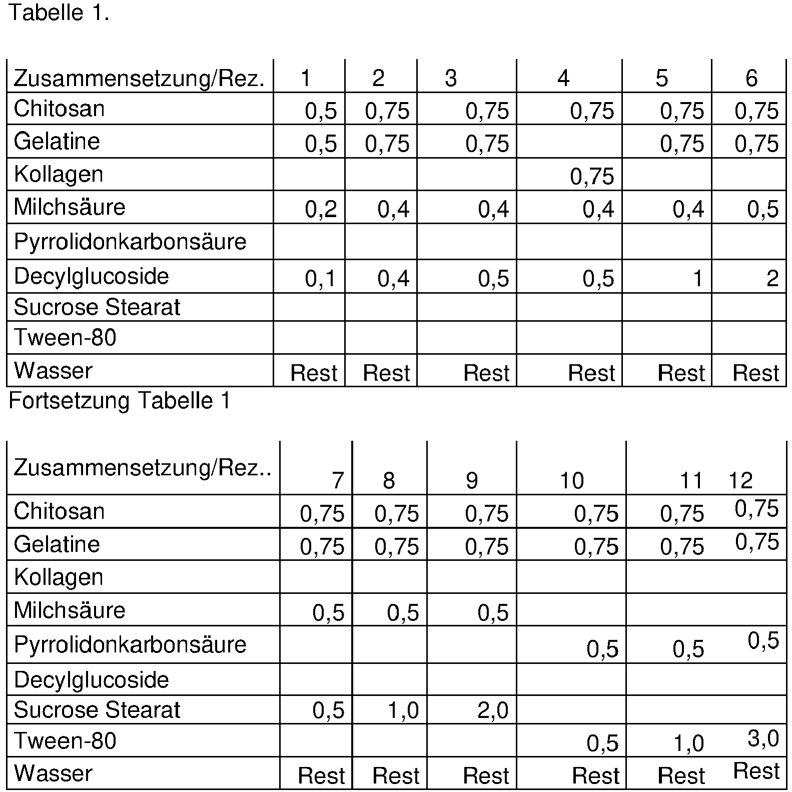

### Beispiel 2: Zubereitungen mit Wirkstoffen (AIP's)

Ähnlich wie in Beispiel 1 beschrieben, wurden Zubereitungen mit Wirkstoffen hergestellt, wobei die APIs während der Herstellung einer Zubereitung oder zu einer bereits fertigen Zubereitung zugegeben wurden, üblicherweise in Form einer API-Lösung. Die Wirkstoffe wurden jeweils bei RT oder höheren Temperaturen von 30 bis 50°C, oder in Anwesenheit von Lösungsvermittlern je nach Wirkstoffbeschaffenheit eingearbeitet. Die einzelnen Wirkstoff-Rezepturen Nr.1-11 sind in Tabelle 2 wiedergegeben:

**Tabelle 2**

| Zusammensetzung | Wirkstoff- Rezeptur Nr. | | | | | | | | | | |
|---|---|---|---|---|---|---|---|---|---|---|---|
| | 1 | 2 | 3 | 4 | 5 | 6 | 7 | 8 | 9 | 10 | 11 |
| Chitosan | 0,75 | 0,75 | 0,75 | 0,75 | 0,75 | 0,75 | 0,75 | 0,75 | 0,75 | 0,75 | 0,75 |
| Gelatine | 0,5 | 0,75 | 0,75 | 0,75 | 0,75 | 0,75 | 0,75 | 0,75 | 0,75 | 0,75 | 0,75 |
| Decylglucoside | 1 | 1 | 1 | 1 | 1 | 1 | 1 | 1 | 1 | 1 | 1 |
| Milchsäure | 0,2 | 0,5 | 0,5 | 0,5 | 0,5 | 0,5 | 0,5 | 0,5 | 0,5 | 0,5 | 0,5 |
| Ethanol | 5 | | 10 | 10 | 5 | 5 | 10 | | | 10 | |

| API | | | | | | | | | | | |
|---|---|---|---|---|---|---|---|---|---|---|---|
| Salicylsäure | 1 | | | | | | | | | | |
| Diclofenac | | 2 | | | | | | | | | |
| Methotrexat | | | 2 | | | | | | | | |
| Minoxidil | | | | 2 | | | | | | | |
| Rose Bengal | | | | | 0,01 | | | | | | |
| Curcumin | | | | | | 0,01 | | | | | |
| Propolis | | | | | | | 0,5 | | | | |
| Harnstoff | | | | | | | | 5 | | | |
| L-Arginin | | | | | | | | | 0,5 | | |
| Clobetasol | | | | | | | | | | 0,01 | |
| Lidocain | | | | | | | | | | | 2 |
| | | | | | | | | | | | |
| Wasser | 91,55 | 95 | 85 | 85 | 91,99 | 91,99 | 86,5 | 92 | 96,5 | 86,99 | 95 |

### Beispiel 3 Verschäumung einer Zubereitung mit einer Applikations-Vorrichtung gemäß Abb. 1

Wie bereits beschrieben, zeigt Abb.1. die wesentlichen Teile einer Applikations-Vorrichtung mit Behälter (2) zur Aufnahme der das Polymer/Tensid/Wirkstoff enthaltenden versprühbaren Zusammensetzung (Z) sowie eine Treibgasquelle (1), hier als externe Sauerstoffzuführungsleitung. Das Treibgas treibt die versprühbare Zusammensetzung bei Betätigung des Applikationssystems durch die Sprühvorrichtung (4), hier eine Laval-Düse. Dabei bildet sich ein Sprühkegel aus feinen Aerosoltropfen, der das poröse Filtermaterial benetzt. Mit der erfindungsgemäßen Zubereitung ist hier eine Liquid/Gas Filtration möglich. Es entsteht eine Gas-Nukleation und damit aufgrund der erfindungsgemäßen Zusammensetzung eine Stabilisierung von Gasbläschen, was zu einem stabilen Schaum (6) führt.

Die Verschäumung/Anwendung der erfindungsgemäßen Zubereitung kann vorzugsweise in folgenden Schritten erfolgen:
- Vernebelung (Aerosolierung) einer Zusammensetzung (Gel) durch eine Düse; (Steuerung durch: Düsenparameter, Druck und Gas- und Liquid- Flow, Strahlform)
- Feine Benetzung eines porösen Materials mit dem Aerosol (Steuerung durch: Aerosol-Parameter, Aerosol-Kegel Parameter, Abstand zu dem Filter, Filtermaterial, Filtereigenschaften);
- Schaumbildung (Gas-Nukleation) durch Benetzung und Stabilisierung von GasBlasen mit der wässrigen Polymer/Tensid-Lösung (Zusammensetzung) (Steuerung durch: Filterfläche, Druck, Flow, Verhältnis Gas/Liquid).

Im Folgenden wurde mit einer Vorrichtung gemäß Abb.1 eine Zubereitung (Nr.5, Tab.1) mithilfe des Sauerstoffgenerators "TDA" der Fa. Meddrop GmbH, Deutschland versprüht. Die Strahlgeschwindigkeit/Partikelkonzentration wurde mittels Dantec Dynamics' Particle Dynamics Analysis (PDA, =Phase Doppler Anemometry) gemessen. Die Aerosol-Partikelgröße wurde mittels Particle Image Velocimetry (PIV)-Systems gemessen. Durch die Aufnahme von einer CCD-Kamera, gekoppelt an einen double pulsed Laser, wurden die Parameter des Strahls bestimmt. U.a. zeigen sie eine sehr homogene Partikelverteilung mit durchschnittlicher Partikelgröße unter 10 µm. Die Ergebnisse sind in Tabelle 3 dargestellt.

**Tabelle 3.**

| Parameter | Wert |
|---|---|
| Das Volumen von appliziertem Liquid (Arzneimittel) | 1 bis 10 ml |
| Sauerstoffdruck | 1 bis 3 Bar |
| Leistung bei einem Druck von | |
| • 2,0 Bar | 20,0 L O₂ /min |
| • 0,5 Bar | 5 L O₂ /Min |
| Strahlgeschwindigkeit (Ausströmungsgeschwind.) | Von 10 bis 50 m/s |
| Strahlausbreitung- Strahlform (2 Bar, 20,0 L O₂/Min) | |
| Strahllänge/Strahlbreite | 25 mm/8 mm |
| Strahlausbreitung- Strahlform (0,7 Bar, 8,0 L O₂/Min) | |
| Strahllänge/Strahlbreite | 8 mm/ 3mm |
| Mittlerer Durchmesser der Aerosolpartikel | ≤ 1 bis 20,0 µm |
| Menge von Sauerstoff in einem Schaum (bei 25°C, 0,5 Bar und 5 L/Min), bei K*≥15 | 30 ml O₂ /ml Schaum |
| Bei K ≥ 40 | 98 mlO₂/ml Schaum |
| Behandelte Hautoberfläche (mit 2 ml Zusammensetzung) | 50 cm² bis 100 cm² |

K* ist der sog. Vervielfältigungskoeffizient, bestimmt durch das Verhältnis zwischen gebildetem Schaumvolumen zum Volumen der Ausgangslösung (K=V Schaum / V _{Liquid}).

Wie ersichtlich, können die aus der erfindungsgemäßen Zubereitung generierten Schäume eine erhebliche Menge von Sauerstoff aufnehmen. Ferner können die Zubereitungen in der geeigneten Applikations-Vorrichtung mit einer Strahlgeschwindigkeit von bis zu 1,0 m/s versprüht werden, ohne dass die Schaumkraft erheblich nachlässt.

### Beispiel 4 Verschäumung einer Zubereitung mit unterschiedlichen Parametern einer Applikations-Vorrichtung gemäß Abb.1.

Wie in Beispiel 3 beschrieben, wurde eine Zubereitung Nr. 5 aus. Tab.1 mit einer Vorrichtung gemäß Abb. 1, Gas-Leistung: Gas (Sauerstoff), P= 0,5 Bar, Flow = 8,0 L/Min verschäumt. Dabei wurden Venturidüsen mit Düsenbohrungen(Durchmesser) von 0,2 bis 0,5 mm, innenmischende Zweistoffdüsen Typ VarioJet® (Lechler GmbH, Deutschland) und Mikrodüsen der Fa. Tetratech Instruments GmbH, Deutschland verwendet. Die Ergebnisse sind in Tabelle 4 dargestellt.

**Tabelle 4.**

| Nr. | Düse Öffnung, Innenmischung | Poröser Körper | Schaumqualität |
|---|---|---|---|
| 1 | 0,2 mm | Gitternetz ¹ | dichter Schaum (K≤ 4) |
| 2 | 0,2 mm | Fritte ² | dichter Schaum (K≤ 6) |
| 3 | 0,2 mm | Polyester-Vlies³ | lockerer Schaum (K≥ 10) |
| | 0,2 mm | Viskose-Vlies³ | lockerer Schaum (K≥ 10) |
| 4 | 0,2 mm | Fritte ² | dichter Schaum (K≤ 6) |
| | 0,2 mm | Vlies³ | dichter Schaum (K≤ 6) |

| | | | |
|---|---|---|---|
| 1. Gitternetz (Stahl) : Edelstahl-Siebgewebe-50 µm (SFT GmbH, Deutschland) 2. Fritte aus Polyethylen (HDPE, Porendurchmesser ca. 20 µm (Carl Roth GmbH, DE) 3. Vlies (aus Polyester und Viskose, Porendurchmesser ca. 10-25 µm (POLO Filtertechnik, DE). | | | |

Wie ersichtlich können erfindungsgemäße Zubereitungen je nach Bedarf unterschiedlich stark oder schwach verschäumt werden, je nach gewünschter Schaumzellengröße.

### Beispiel 5 Steuerung des Bläschenverteilung einer anmeldungsgemäßen Zubereitung / Flow Rate

Die Schaumeigenschaften (Schaummorphologie, ermittelt z.B. durch Schaumstabilität, Schaumzellengröße und andere Parameter) können erfindungsgemäß gesteuert werden von einem homogenen kleinzelligen oder grobzelligen Schaum zu einem heterogenen Mischschaum mit breitem Spektrum von Zellengrößen je nach Anwendungsbereich und/ oder Ziel.

Im folgenden Beispiel wurde in einer Vorrichtung gemäß Abb.1 mit Filtermaterial Nr. 3 aus Tab 3 (Polyester) eine Zubereitung gemäß Beispiel 1 (Tab.1, Nr.5) verschäumt. Als Gasquelle wurde Sauerstoff (95 Vol.% O₂) gewählt (Sauerstoffgenerator: "TDA" der Fa. MedDrop GmbH). Eine Düse der Fa. Lehler GmbH mit 0,2 mm Durchmesser wurde verwendet.

Es wurde der Blasendurchmesser (Schaumzellengröße) des erhaltenen Schaumes in µm mittels Digital Mikroskop (Fa. Di-Li, Deutschland) ermittelt.

Die Ergebnisse sind in Abb. 2 graphisch dargestellt. Wie ersichtlich, steigt bei einem konstanten Gas-Flow in einem breiten Spektrum von 0,1 L/Min bis 3,0 L/Min der Blasendurchmesser des gebildeten Schaums mit steigendem Liquid-Flow. Der Bläschendurchmesser beträgt hier beispielsweise 440 µm bei einem Liquid-Flow von 0,5 L/Min und ca. 200 µm bei 1,7 ml/min. Bei einem konstanten Liquid-Flow verkleinern sich die Bläschen mit steigendem Gas-Flow, z.B. bei einem Liquid-Flow von 2,0 ml/min von 300 µm (bei ca. 0,5 L O₂/min) bis 100 µm (bei ca.0,9 L O₂/min).

Mit der erfindungsgemäßen Zubereitung kann somit die Bläschenverteilung je nach Anforderung gesteuert werden.

### Beispiel 6 Steuerung der Bläschenverteilung einer anmeldungsgemäßen Zubereitung / Tensidgehalt

Im Folgenden wurde die Schaumstabilität in Abhängigkeit von der zugegebenen Tensidmenge in einer Zubereitung gemäß Beispiel 1 (Nr. 4 bis 6, Tab.1) mit steigender Tensidkonzentration untersucht.

Die Zubereitung wurde variiert durch Änderung der Tensidmenge von 0,5% bis 2,0 % (Gew.%) Decylglucosid. Die Zubereitungen wurden mittels einer Vorrichtung gemäß Abb. 1 und einem Filter Nr.3 aus Polyester gemäß Tab. 4, Beispiel 4 versprüht.

Die Ergebnisse sind in Abb. 3 dargestellt.

Aus Abb. 3 folgt, dass mit steigender Tensid-Konzentration von 0,5% bis 2,0% in einer Zusammensetzung die Bläschen sich verkleinern und eine bessere Homogenität aufweisen. Je nach beabsichtigtem Ziel kann auch die Schaumstabilität beeinflusst werden: mit 0,5% Decylglukosid kollabiert ein Schaum in 3-4 Stunden (Schaum 2), während ein Schaum auf Basis von 2,0%-igem Tensid mehr als 15 Stunden stabil bleiben kann (Schaum 3). Dadurch besteht die Möglichkeit, die Schaumstrukturen zu beeinflussen, in gewünschter Richtung zu steuern und eine gewünschte Schaumstabilität je nach Anforderung zu gewährleisten.

### Beispiel 7 Schaum Stabilität im Zusammenhang mit der Chitosan/Gelatine Konzentration:

Im Folgenden wurde die Schaumzellenverteilung einer Zubereitung gemäß Beispiel 1, Basisrezeptur mit einer Chitosan / Gelatine-Konzentration von insgesamt 0,5% (A), 1,0% (B) und 1,5 % (C), (Nr.5, Beispiel 1) bestimmt. 2 Stunden nach ihrer Herstellung wurde die Morphologie der Schäume A bis C mit Hilfe eines Digital-Mikroskops gemessen. Die Ergebnisse sind in Abb.4 dargestellt. Wie ersichtlich, korreliert die Vergrößerung der Blasen (Schaumzellen) u.a. mit der Viskosität der Formulierung (in erster Linie ermittelt durch % Biopolymer): je höher die Konzentration des Polymergemischs, desto langsamer ist die Disproportionierung (Koaleszenz) der Schaumbläschen, ermittelt durch Drainage.

Es zeigte sich, dass die Zubereitung mit 0,5% Polymer einen Blasenmittelwert von ca. 780 µm, bei 1,0 % Polymer 560 µm und bei 1,5% nur 110 µm Durchmesser ermöglicht.

Die Ergebnisse aus den Beispielen 6 und 7 zeigen, dass durch einfache Variation einiger Parameter die Möglichkeit besteht, die Beschaffenheit des Schaumes in einem gewünschten Ausmaß zu beeinflussen und somit Zubereitungen mit jeweils gewünschten Eigenschaften flexibel herzustellen.

### Beispiel 8: Auf die Haut applizierter Schaum: Sauerstoffpenetration in die Haut (tpO₂)

Für die Vermessung der Sauerstoffdiffusion in die Haut wurde Zubereitung Nr.3, Tab.1 untersucht.

Zu den Messungen wurden gesunde Probanden ohne Hautverletzungen/Hautkrankheiten ausgewählt. Während der Messungen mussten die Probanden auf Kaffee, Zigaretten und Alkohol verzichten. Die Messungen fanden in einem klimatisierten Raum (T=20-23°C) statt.

Die Haare wurden abrasiert und die Haut wurde mit einem Reinigungsmittel (Bacillol® 25, Fa. Bode Chemie, Deutschland) vorab gereinigt und an der Luft getrocknet. Die Kleberinge für zwei Elektroden wurden an der Innenseite des Unterarmes 3 cm vom Ellbogen (1 . Kontrolle) und 6 cm vom Ellbogen (2. O₂-Schaum) aufgeklebt. Der untersuchte Schaum wurde auf die Haut (Kleberinge 2) gegeben und leicht einmassiert. Nach 10 Minuten Einwirkung (mit bzw. ohne Sauerstoffschaum- Kleberinge 1) wurde der Rest des verbliebenen Schaumes vorsichtig mit einem Tuch entfernt. Zu allen Kleberingen wurde Elektrolytlösung (Radiometer, Dänemark) zugegeben und die Elektroden angeschlossen.

Der Nachweis der Sauerstoffpenetration in die Haut wurde mittels TCM 4 (Radiometer, Dänemark) durch Vermessung des transcutanen Sauerstoffpartialdrucks erfasst. Die Ergebnisse sind in Abb. 5 dargestellt.

Die Messung wurde bei 37°C und 45°C jeweils nach Erreichen von stabilen Werten durchgeführt.

Die mit dem Schaum behandelte Haut zeigte im Vergleich zu der Kontrolle bereits nach 7 Minuten erhöhte pO2 -Werte in der Haut. Die Werte stiegen im Laufe der Zeit weiter, und nach etwa 15-20 Minuten erreichten sie ihr Maximum pO₂ -Wert der Schaum um 25 % zur Kontrolle

Überraschend wurde gefunden, dass die aus den erfindungsgemäßen Zubereitungen generierten sauerstoffhaltigen Schäume in der Lage sind, erhebliche Mengen an Sauerstoff durch die Haut zu transportieren. Dadurch steigt der pO₂-Wert um ca.10-20 mm Hg, wie in Abb.5 dargestellt. Im weiteren Verlauf ist zu erkennen, dass diese zusätzliche O₂-Menge den O₂-Verbrauch der Zellen (bereits nach ca. 30 Minuten) aktiviert und dadurch die dort ablaufenden biochemischen Prozesse aktivieren.

Es wird angenommen, dass mit der erfindungsgemäßen Zubereitung aus vor allem Chitosan und Gelatine und nicht-ionischem Tensid nicht kovalente Assoziationen mit erhöhtem O₂-Adsorptions- und Diffusionspotential durch die Haut entstehen.

### Beispiel 9 Verträglichkeitstest / Hautapplikation

Zur Ermittlung der Hautverträglichkeit des Schaumes werden physikalische Eigenschaften (Hautadhäsion, Verteilung) und dermatologische Parameter (Hautreaktionen, sowie Juckreiz, Allergie oder Akzeptanz beim Patienten) herangezogen.

Das Verhalten eines Schaums, erhalten durch Versprühen einer Zubereitung gemäß Nr. 5 Tabelle 1 (Beispiel 1) mittels einer Vorrichtung (Abb.1 und Nr.3 Beispiel 4) wurde auf einer Humanhaut an freiwilligen Probanden (insgesamt 12 Personen -5M/7F) untersucht. Die Messung der TEWL erfolgte wie nachfolgend angegeben:
Die Barrierefunktion jeder Hautprobe wurde durch Messung ihres transepidermalen Wasserverlustes (TEWL) bewertet. Dies erfolgte durch Verwendung eines Tewameters TM300 (Courage & Khazaka GmbH, Köln, Deutschland). Stabile TEWL-Meßwerte wurden aufgezeichnet. Die für die Studien verwendeten Hautproben zeigten einen TEWL von 9,5 ± 1,40 g / m² / h (Mittelwert ± SD), während die mit der aufgeschäumten Zubereitung behandelten Hautproben einen TEWL von 8,52 ± 1,12 g / m² / h aufwiesen (Mittelwert ± SD). Diese Werte unterscheiden sich nicht signifikant voneinander (t-Test: P = 0,38). Darüber hinaus sind diese TEWL-Werte niedrig genug, um anzuzeigen, dass alle Testhäute intakt waren ohne negative Beeinträchtigung auf ihre Hautbarrierefunktion.

In der Tab. 5 sind die Ergebnisse zusammengefasst.

**Tabelle 5. Verhalten eines Schaumes auf der Humanhaut. (Schaum aus Tab.1, Nr.5).**

| Parameter | Bewertung | Kommentar |
|---|---|---|
| Abdeckung | Sehr gut verteilt | |
| | | |
| Hautadhäsion | sehr gut | Haftet langzeitig auf der Haut |
| Verteilung (mit Spachtel) | Gut, regelmäßig | |
| TEWL | 8,52 ± 1,12 g/m2/h | Vergleichbar mit intakter Haut |
| Hautreizung | keine | Epikutantest* |
| Allergischer Ausschlag | keine | Epikutantest |

| | | |
|---|---|---|
| Epikutantest* wurde an 26 Probanden (15F/11 M) durchgeführt, gemäß etablierter Methode. | | |

Hieraus folgt, dass die erfindungsgemäße Formulierung unbedenklich und sicher ist bei der Hautapplikation. Ebenfalls zeigt sie eine sehr gute Hautadhäsion, wobei der Transkutane Wasserperspirationsprozess (TEWL) nicht negativ beeinträchtigt wird.

### Beispiel 10 Infizierte Wunden, Wundheilung: Antibakteriell wirkende Sauerstoff-Schaumzubereitung

In einer experimentell erzeugten eitrigen Wunde wurden die antimikrobiellen und reparativen Regenerationsprozesse in erster Linie durch Beeinflussung der frei-radikalischen Oxidationprozesse mit Hilfe des erfindungsgemäßen Schaums (Nr.3, Tab.1. Beispiel 1) untersucht.

Die Studie wurde an 60 weißen männlichen geschlechtsreifen Wistar-Ratten mit einem Körpergewicht von 180-230 g durchgeführt. Drei Gruppen davon waren 25 -Verum, 25 - Kontrollen/Wunden, 10-intakte Tiere. Das Modell einer vollschichtigen Wunde wurde unter Anästhesie im Rückenbereich durch Exzision des Hautlappens mit subkutaner Faser in Form eines Quadrats in der Größe von 20 × 20 mm gebildet.

Die Wundinfektion wurde an 50 Tieren mit Hilfe eines Gaze-Tampons (mit einem Gewicht von 0,5 g), der mit einem von Patienten mit aus eitrigen traumatischen und postoperativen Wunden isoliertem antibiotika-resistentem Staphylococcus aureus in einer Dosis von 2 × 10 ⁶ KBE / ml induziert. Am 2.-3. Tag entstand eine infizierte eitrige Wunde in Begleitung von entzündlichen Prozessen.

Geeignete Tierversuche wurden gemäß den geltenden Tierschutzgesetzen genehmigt und in Einklang mit der Leitlinie zur guten klinischen Praxis, ICH E6 (R1) durchgeführt. Die Haltung der Tiere, deren Nahrung und Pflege wurde nach dem Tierschutzgesetz vorgenommen.

Die Behandlung erfolgte mit einem erfindungsgemäßen O₂-Schaum (gemäß Beispiel 1 Rezeptur Nr. 3, Tab.1), ohne Einsatz von zusätzlichen API, und Schaumapplikationssystem (Beispiel 4, Nr.3), angeschlossen an den Sauerstoffgenerator "TDA" (Fa. MedDrop, Deutschland). Die Zubereitung (2 ml Schaumgel) ergibt ca. 100 cm³ Schaum. Dieser Schaum wurde in die Wunden innerhalb von 2 Minuten appliziert und danach zusätzlich mit Sauerstoffgas aus dem Applikator 5 Minuten begast.

Die Behandlungen wurden ab dem dritten Tag nach Verwundung einmal täglich an jedem zweiten Tag durchgeführt.

Tiere in der Kontrollgruppe wurden mit einer 0,9%-igen isotonischen Lösung von Natriumchlorid behandelt.

Der Wundheilungsprozess wurde beurteilt durch die Anwesenheit und die Art der entzündlichen Reaktionen (in erster Linie verursacht durch Entstehung von freien Radikalen), und den antioxidativen Status der Tiere. Biochemische Laboruntersuchungen wurden im Laufe des Heilprozesses durchgeführt. Es wurde der Gehalt an Malondialdehyd (in der Reaktion mit Thiobarbitursäure) im Serum zur Aktivitätsbewertung der Lipoperoxidationsprozesse gemäß der etablierten ELISA-Labormethode bestimmt.

Die reparativen Prozesse wurden durch den Zustand der Ränder und des Bodens der Wunde, den Zeitpunkt der Reinigung der Wunden von nekrotischem Gewebe, das Auftreten von Granulationen und Epithelialisierung ermittelt. Das Wundausmaß wurde durch Vermessung anhand seiner Größe am den 3., 5., 7., 12., 14 und 21. Tag dokumentiert (Fotoaufnahme). Der Wundzustand und die biochemischen Parameter wurden regelmäßig gemessen und beurteilt.

Die statistische Verarbeitung von den generierten Daten wurde mit Hilfe des Medstat-Programms, für die Verarbeitung von medizinischen und biologischen Beobachtungen durchgeführt.

Die erzogenen eitrigen Wunden wurden charakterisiert durch Hyperämie am Rand und Boden der Wunden. Am dritten Tag gab es einen eitrigen Ausfluss. In diesem wurden Bakterien von S. aureus in einer Menge von 3 x 10⁸ KBE / ml bakteriologisch nachgewiesen. Die Tiere waren lustlos, unordentlich, das Essen wurde oft abgelehnt, so dass sie Gewicht verloren.

Bei Tieren mit einer eitrigen Wunde vor der Behandlung wurden erhöhte Werte von Lipidperoxiden festgestellt, was als Kriterium für die Belastung des anti-oxidativen Systems gilt. Bereits am 3.Tag wurde ein erhöhter Wert der Konzentration an Malondialdehyd (4,51 ± 0,23 µmοl/l), als Begleiter der Lipidperoxidation in den Kontroll- und Verum-Gruppen, im Vergleich zu gesunden Ratten (2,47 ± 0,17 µmοl/l) beobachtet. Dies weist auf die wesentliche Intensivierung von entzündlichen Prozessen, die durch Lipidperoxidation hervorgerufen wurden, hin. Eine weitere Bestätigung einer Belastung des Antioxidantien-Systems war erkennbar an der Abnahme der Aktivität des körpereigenen Oxidase Ceruloplasmins auf 18,25 ± 0,76 i.e., im Vergleich zu intaktem Tier (25, 24 ± 0,50 i.e.).

Nach der Behandlung sank der Gehalt an Malonsäuredialdehyd am 5. Tag auf 3,55 ± 0,20 µmοl/l und am 10.Tag bis auf 2,71± 0,64 µmol/l. Zu dieser Zeit gab es auch eine positive Veränderung in dem Zustand des Antioxidantien-Systems mit steigender Aktivität von Ceruloplasmin auf 23,18 ± 0,52 i. e.

In der Kontrollgruppe (Behandlung mit 0,9%-iger NaCl) stiegen am 14. Beobachtungstag die Malodialylaldehyd-Werte weiter bis 4,90 ± 0,26 µmol/l, und die Aktivität von Ceruloplasmin sank auf 16,46 ± 0,68, was eindeutig auf eine weitere Intensivierung von oxydativen Prozessen hindeutet.

Die biochemische Untersuchungen haben eine gute Korrelation mit der nachgewiesenen Anzahl an bakteriellen Infektionen. In der Verumgruppe am 5. Tag betrug die Anzahl von S. aureas 2x10⁶ KBE/ml , am 7.Tag -2x10⁴ KBE/ml und am 12.Tag war sie nicht mehr nachweisbar. In der Kontrollgruppe betrug die Anzahl von Bakterien bis zum 12. Tag 5 x10⁷ bis 3x10⁸ KBE/ml.

Bei den Ratten der Verum-Gruppe war bereits am 5. Tag nach der Schaum-Applikation eine Verbesserung des Wundzustands zu beobachten Die Wundoberflächen zeigten eine klare Grenze und eine sichtbare Wundkontraktion zur Vergleichsgruppe (Tab.6).

**Tabelle 6. Wundkontraktion im Laufe der Behandlung (M±m, mm²)**

| Tag | Kontrolle, mm² | Verum Gruppe, mm² |
|---|---|---|
| 1 | 390,5±5,3 | 386,4±4,9 |
| 3 | 385,4±5,0 | 362,5±5,3 |
| 5 | 382,3±4,7 | 313,4±6,1 |
| 7 | 367,2±4,2 | 248,6±3,7 |
| 8 | 300,5±3,9 | 152,,9±4,8 |
| 12 | 284,4±3,4 | 55,1±4,4 |
| 14 | 252,4±2,6 | 25,5±5,4 |
| 21 | 199,7±5,9 | vollständige Heilung |

Aus der Tab.6 ist eine wesentliche Beschleunigung der Wundheilung bei der Schaum-Gruppe im Vergleich zur Kontrollgruppe zu erkennen. In der Schaum-Gruppe gab es am 7. Tag eine Abnahme in der Wundfläche um das 2,6 fache, ohne Entzündungszeichen in der Wunde zu messen. Unter dem Einfluss des Schaumes wurde bereits am 10. Tag nach der Bildung von einer vollinfizierten Wunde bei einzelnen Vertretern der Experimentalgruppe eine fast vollständige Epithelisierung und Wiederherstellung der Haarbedeckung festgestellt. Bei den übrigen Vertretern der Experimentalgruppe war eine vollständige komplette Epithelisierung am 14 - 21. Tag zu sehen.

### Beispiel 11: IOT (Integrale Sauerstoff Therapie)

Die positiven Effekte der erfindungsgemäßen Zubereitungen zeigen sich auch bei der Wundbehandlung von Patienten in multizentrischen Studien (mit zwei oder mehreren) Wunden an den Extremitäten (Bein/Fuß/Arm).

Die Behandlung erfolgte mit einer erfindungsgemäßen Zubereitung gemäß Beispiel 1 Rezeptur Nr. 3, Tab.1, ohne Einsatz von zusätzlichen API, und Einsatz einer Applikations-Vorrichtung gemäß Nr.3, Beispiel 4, angeschlossen an den Sauerstoffgenerator-"TDA"

(Fa. MedDrop, Deutschland). Die Zubereitung (2 ml Schaumgel) ergab ca. 100 cm³ Schaum. Dieser Schaum wurde in die Wunden innerhalb von 2 Minuten appliziert und danach zusätzlich mit Sauerstoffgas aus dem Applikator 10 Minuten begast. In der Kontrollgruppe wurden kommerzielle Wundauflagen auf Alginat-Basis (Aquacell® Convatec GmbH, Deutschland), Kollagen-Basis (Suprasorb Kollagen, Lohman & Rauscher, Deutschland) gemäß Gebrauchsinformation eingesetzt.

Die Behandlungen wurden ab dem ersten Tag einmal an jedem zweiten Tag durchgeführt. Die Ergebnisse sind in Tabelle 7 zusammengefasst.

**Tabelle 7**

| Diagnose | Behandlung/Wundauflage | Anzahl der Patienten | Erste Granulation | Ende der Epithelisierung | Nebenwirkungen |
|---|---|---|---|---|---|
| posttraumatische Wunden (u.a. nach autologer Hautplastik) | Erfindung | 21 | am 3. -5. Tag | am 8. - 9. Tag | keine |
| posttraumatische Wunden (u.a. nach autologer Hautplastik | kommerziellen WA Aquacell® | 14 | am 4. -6. Tag | am 12. - 15. Tag | allergische Dermatitis (2) |
| chronische Wunden durch CVI* | Erfindung | 13 | am 5. - 6. Tag | 50%-ige Verkleinerung der Wunde am: 30.-35. Tag | keine |
| chronische Wunden durch CVI* | kommerzielle Wundauflagen Suprasorb | 12 | am 8. - 10. Tag | am 40. Tag | allergische Dermatitis (3) |

| | | | | | |
|---|---|---|---|---|---|
| CVI*- chronisch venöse Insuffizienz | | | | | |

Aus Tab. 7 ist gut zu erkennen, dass die erste Granulation in der O₂-Schaumgruppe 1 bis 3 Tage schneller stattfand als bei kommerziellen Wundauflagen. Ebenso verlief die komplette Epithelisierung um ca. 30-35% schneller als bei kommerziellen Wundauflagen. In Rahmen der IOT-Behandlung wurden keine Nebenwirkungen beobachtet. Im Gegensatz dazu wurden bei den kommerziellen Wundauflagen allergische Hautreaktionen beobachtet.

Mit den erfindungsgemäßen Zubereitungen ist somit eine effiziente Anwendung möglich. Aufgrund der Flexibilität der Zubereitung sowohl bzgl. der Wirkstoffe als auch im Hinblick auf Anwendungsparameter wie hohe/niedrige Schaumdichte (Blasengröße) lässt sich auch auf einfache Weise eine Konsekutivanwendung in einem Modus vornehmen wie z. B. bei Wundheilungen zunächst mit Wirkstoff (z. B. Antibakteriell) als Schaum, danach ohne Wirkstoff, versprüht, erneut z.B. mit Pflegestoffen als Schaum, versprüht. Da Sauerstoff in hoher Konzentration in die Präparation (insbesondere 5 bis 10-mal höher, im Vergleich zu den konventionellen Produkten) eingetragen werden kann, ist eine erleichterte Diffusion hiervon in die tieferen Gewebezellen möglich und damit eine bessere Wiederherstellung von Gefäßen und Kapillaren und insgesamt eine Beschleunigung in allen Wundheilungsphasen möglich (siehe Beispiel 8). Aufgrund der Einsatzflexibilität ist insgesamt eine Kostenreduktion möglich.

### Beispiel 12 Behandlung von Onychomykose mit Curcumin enthaltender verschäumter Zubereitung

Die photodynamische Therapie (PDT) ist eine nichtinvasive Technik zur Therapie von Problemhaut, u.a. Onychomykose durch die Assoziation eines photosensibilisierenden Mittels und einer Lichtquelle mit bestimmter geeigneter Wellenlänge. Sauerstoff, der durch einen Photosensibilisator und Licht aktiviert wird, generiert die reaktiven Sauerstoffspezies, die Infekte abtöten und dadurch die Heilung ermöglichen.

Zwei Patienten (Fall 1: männlich, 47 Jahre alt, Fall 2: weiblich, 65 Jahre alt) mit Fußpilzerkrankungen wurden ärztlich und mikrobiologisch untersucht. Die Ergebnisse waren positiv bzgl. der Laborkulturen, die auf das Vorhandensein von Pilzen hinwiesen. Beide Patienten hatten Läsionen für etwa 5 Jahre.

Der Nagel wurde unmittelbar vor der Applizierung für 2 Minuten mit 25% Harnstoffgel abgedeckt. Alle Behandlungen wurden mit Curcumin-haltiger verschäumter Zubereitung gemäß Beispiel 2 (0,01% Curcumin, Rezeptur Nr. 6,Tabelle 2) durchgeführt. Der Schaum wurde topisch mit einem Applikator gemäß Beispiel 3 wie in Beispiel 11 beschrieben aufgetragen. Als Gasgenerator wurde ein Sauerstoffgenerator "TDA" (Fa. MedDrop, Deutschland) eingesetzt. Der O₂-Schaum wurde anschließend einer Licht-Bestrahlung unterzogen.

Nach ca. 3 Minuten nach Schaumauftragung wurden die Patienten mit Onychomykose für 20 Minuten mit einem LED - System (450 nm, 100 mW / cm², [Philips BlueControl PSK0202, Philips, Deutschland]) behandelt. Zehennägel wurden mit Gesamtstrahlung von 120 J / cm² belichtet.

Jede Behandlung wurde mit fotografischen Bildern und mikrobiologischen Untersuchungen dokumentiert.. Es wurden insgesamt 6 PDT-Sitzungen durchgeführt.

Ergebnis:
Bei Patient 1 wurde nach 5 PDT-Sitzungen und bei Patient 2 nach 6 PDT-Sitzungen eine vollständige Heilung festgestellt. Die Ergebnisse sind in Abb. 6 dargestellt.

### Beispiel 13 Anwendung bei Problemhaut (Psoriasis): Therapie mit Methotrexathaltigen Schaum.

Zum Nachweis der positiven Effekte bei Psoriasis Behandlung unter Anwendung einer Methotrexat (MTX)-haltigen (2%) Zubereitung gemäß Tab.2, Nr.3, und Sauerstoffgenerator-TDA aus Beispiel 4, Nr.3 (MedDrop GmbH, Deutschland) auf Abheilung von Psoriasis wurde eine multizentrische Parallelgruppen-Studie an 26 Patienten mit Diagnose Psoriasis-Plaque-Typ durchgeführt. Die Patientengruppe (männlich/weiblich) war zufallsverteilt mit je 13 Patienten pro Gruppe: MTX-Schaum-Gruppe und MTX-Gel-Gruppe (Methotrexat in einer Konzentration 1,5 % in Basiscreme DAC- ist ein Apotheken-Präparat). Die Behandlung wurde über einen Zeitraum von 14 Wochen durchgeführt mit drei Behandlungen pro Woche.

Das Ausmaß und die Schwere der Hautveränderungen wurden mit Hilfe des psoriasis and severity index (PASI) ausgewertet. Das Erythem, die Infiltration und die Schuppung an den Körperteilen wurden ausgewertet. Die generelle Einschätzung wurde auf einer Scala von 0 bis 5, von einer complet clear Haut bis nicht geheilt, eingesetzt.

Für die Auswertung von Messwerten wurde der Student-t-Test eingesetzt. Die Ergebnisse wurden als p-Wert berechnet. Mit Signifikanzniveau p=0,05 gilt das Ergebnis als statistisch signifikant.

Ergebnis:
Nach 15-wöchiger Behandlung wurde bei 22 Patienten (87%) eine komplette Abheilung der psoriatischen Herde beobachtet.

Nach 6 Wochen wurden die PASI-Werte um 40% (MTX-Gel-Luft) und bzw. 60% (MTX-Schaum plus reiner Sauerstoff) verbessert. Nach 14 Wochen wurden die PASI-Werte um 59% (MTX-Gel-Gruppe) und 88% (MTX-Schaum plus Sauerstoff) verbessert.

Die Ergebnisse sind in Abb. 7 dargestellt.

### Beispiel 14 Kosmetische Anwendung auf Haare mit Wirkstoff Minoxidil.

Eine Zubereitung gemäß Rezeptur Nr.4, Tab.2 Minoxidil (ML-haltig) wurde als Schaum aus 1,5 ml ML-Zubereitung wie in Beispiel 12 beschrieben appliziert und zusätzlich mit Sauerstoff im Laufe von 15 Minuten begast.

Als Vergleich wurde ein kommerzielles ML-haltiges Präparat (Regaine, 2% ML, Johnson& Johnson) gemäß Beipackzettel aufgetragen.

In die zweiarmige, randomisierte Studie wurden 60 Frauen einbezogen, nämlich gesunde Frauen ab 18 Jahre alt mit einem Befund der AGA, basierend auf einer Abnahme der Haardichte auf dem Oberkopf (Salvin Grad 3-6). Weiteres Einschlusskriterium war eine Haardichte kleiner gleich 220 Haare/cm², gemessen mit dem TrichoScan.

Das Untersuchungsareal (ca. 2 cm²) wurde identifiziert, rasiert und mit schwarzer Haarfarbe eingefärbt und markiert. Dieses wurde mittels TrichoScan auf die Anzahl der Haare(n)/cm² (oder Haardichte) untersucht. Die Analyse des TrichoScan erfolgte an einem Kamera-PC (TrichoScan® Research, Freiburg, http://trichoscan.com). Für die TAHC (n/cm²) wurden für jede Probandin, für jede Visite, die Haare mit einem Durchmesser von 30 µm zusammenaddiert.

Die Ergebnisse sind in Abb. 8 dargestellt. Wie ersichtlich, war die erfindungsgemäße Zubereitung (O₂-Schaum 1,5% ML) bei einmal täglicher Anwendung effizienter als Regaine (2% ML) Die kosmetische Akzeptanz beider Präparate war gut, jedoch waren die Probandinnen des Schaums signifikant zufriedener bezüglich Haarpflege und Haarstyling.

## Patentansprüche

1. Einen Bio-Polymerschaum generierende und versprühbare Zubereitung (Z) auf Chitosan / Eiweiß- Basis, **dadurch gekennzeichnet, dass** sie besteht aus:
a) 0,1 bis 5,0 Gew. % Chitosan, MM 70.000 bis etwa 2.000.000 Da (70 KDa bis 2000 KDa), oder deacetyliertes Chitosan, MM 70.000 bis etwa 2.000.000 Da (70 KDa bis 2000 KDa) mit einem Deacetylierungsgrad von 60 bis 98 %,
b) 0,1 bis 5,0 Gew.%, Eiweiß, ausgewählt aus Kollagen Typ I,II, III, oder Mischungen hiervon, Kollagenhydrolysat oder Gelatine mit mittlerem MW von 20 kDa bis 60 kDa, oder Mischungen hiervon,
wobei Chitosan und Eiweiß im Verhältnis 4:1 bis 1:4 vorliegen;
c) 0,1 bis 5,0 Gew.% Säure, ausgewählt aus Mono- oder Bi- oder Trifunktionellen Carbonsäuren, ausgewählt aus Essigsäure, Milchsäure, Äpfelsäure, Bernsteinsäure, Malonsäure, Fumarsäure, Ascorbinsäure, Propionsäure, Glutaminsäure, Salicylsäure, Pyrrolidoncarbonsäure, oder Kombinationen zweier oder mehrerer dieser Säuren;
d) 0,1 bis 5,0 Gew. % eines oder mehrere nichtionischer Tenside, ausgewählt aus C₆ -C₂₂ Alkylglucosiden, C₆ -C₂₂ -Alkylpolyglceriden, Polyglyceryl-C₆ -C₂₂- Fettsäureestern, polyoxylierten Polyglyceriden, polyoxylierten C₆ -C₂₂ -Fettsäuren, polyoxylierten C₆-C₂₂ -Fettalkoholen, C₆ -C₂₂ - Fettalkoholpolyglykolethern, oder Mischungen hiervon,
e) 0,01 bis 20 Gew. % Hilfsstoffe, ausgewählt aus Viskositätsregulatoren, Antioxidantien, Farbstoffen, Konservierungsmitteln, pH-Regulatoren, Lösungsvermittlern oder Mischungen hiervon;
f) 0 bis 20 Gew.%, insbesondere 0,01 bis 20 Gew. % Wirkstoffe, ausgewählt aus kosmetischen Wirkstoffen, pharmazeutischen Wirkstoffen oder Mischungen hiervon;
g) 60 bis 95 Gew % Wasser oder wässrige Lösung,
wobei die Zubereitung Strukturen mit einer homogen einstellbaren differenzierbaren Schaumzellengröße im Bereich von 0,01 bis 2,0 mm aufweist.

2. Zubereitung nach Anspruch 1, **dadurch gekennzeichnet, dass** sie eine Aufnahmekapazität für Gas, Sauerstoff-haltiges Gas oder Sauerstoffgas im Schaum von 10 bis 100 ml O₂ haltiges Gas oder O₂ pro 1 bis 3 ml Zubereitung aufweist.

3. Zubereitung nach Anspruch 1 oder 2, **dadurch gekennzeichnet, dass** sie 0,1 bis 5 Gew. % einer Biopolymermischung aus deacetyliertem Chitosan, MM von 300 kDa bis 800 kDa, und Gelatine vom Bloomwert von 200 bis 300 aufweist.

4. Zubereitung nach einem der Ansprüche 1 bis 3, **dadurch gekennzeichnet, dass** das Tensid ausgewählt ist aus einem oder mehreren C₁₀ -C₂₂- Alkylglycosiden, C₆ -C₂₂-Alkylpolyglykosiden, Sacharose- (Sucrose-)-C₆ -C₂₂-Alkyl-estern, C₆ -C₂₂- Fettalkoholpolyglykolethern oder Mischungen hiervon.

5. Zubereitung nach Anspruch 4, **dadurch gekennzeichnet, dass** als Tensid Decylglucosid vorliegt.

6. Zubereitung nach einem der Ansprüche 1 bis 5, **dadurch gekennzeichnet, dass** bis zu 45 Gew.%, bezogen auf die Gesamtmenge an Chitosan ersetzt oder ergänzt ist durch Biopolymere aus der Gruppe, umfassend Hyaluronsäure, Chondroitin, Chondroitin-4-sulfat, Chondroitin-6-sulfat, Dermatansulfat, Keratansulfat, Heparinsulfat und Heparin, oder geeignete Mischungen hiervon, Alginat, Dextran, Xanthan, Alginsäure, Agar (Galactose-Polymer), Carragenan, Carboxymethyl--, Isopropylcellulose, α-Amylose, Amylopektin oder Mischungen hiervon, wobei die Gesamtmenge an Polysaccharid wie in Anspruch 1 angegeben ist.

7. Zubereitung nach einem der Ansprüche 1 bis 6, **dadurch gekennzeichnet, dass** bis zu 45 Gew.%, bezogen auf die Gesamtmenge an Eiweiß ersetzt oder ergänzt ist durch Eiweiße aus der Gruppe, umfassend Albumin, Lactoglobulin, Fibronektin und Mischungen hiervon, wobei die Gesamtmenge an Eiweiß wie in Anspruch 1 angegeben ist.

8. Zubereitung (Z) gemäß einem der Ansprüche 1 bis 7, **dadurch gekennzeichnet, dass** sie mizellenartige Biopolymer-, vor allem Chitosan-Tensid- Strukturen aufweist.

9. Zubereitung nach einem der Ansprüche 1 bis 8, **dadurch gekennzeichnet, dass** als kosmetischer Wirkstoff Vitamin A, B, E, C, Moisturizer, Antioxidantien, Peelingmittel, Depilatoren, antipigmentierende Wirkstoffe , Aloe Vera, Centella asiatica, Lavendelöl, Rosmarinöl, Citrusöl, Orangenöl, Minzöl, Ylang Ylang, Jasminöl, Hyaluronsäure, Natriumalginat, Haarwuchsmittel oder Mischungen hiervon vorliegen.

10. Zubereitung nach einem der Ansprüche 1 bis 9, **dadurch gekennzeichnet, dass** ein Pharmazeutischer Wirkstoff aus der Gruppe aus Antiallergenen, Antibiotika, Antiseptika, Virustatika, Antimykotika, Zytostatika, Antiplogistika, Immunsuppressiva, Immunmodulatoren, Immunstimulantien, Antipsoriatika, Keratolytika, Antischmerzmittel, Lokalanästhetika, Hormone, antineoplastische Wirkstoffe, Zell-und Zellorgane regulierende/unterstützende (Wachstumsfaktoren, Zytokine), wundheilende Stoffe oder Mischungen hiervon vorliegen.

11. Zubereitung nach einem der Ansprüche 1 bis 10, **dadurch gekennzeichnet, dass** sie frei ist von ionogenen Tensiden, quaternären Polyacrylat-Polymeren oder quaternisierten Celluloseverbindungen oder Mischungen hiervon.

12. Zubereitung gemäß einem der Ansprüche 1 bis 8, 10,11 zur Anwendung bei der topisch therapeutischen Behandlung geschädigter oder Problemhaut, bei der Behandlung von chronischen Wunden, infizierten Wunden, Verbrennungen, rheumatischer Arthritis, Schmerzlinderung, Haut-und Fußpilz, Allopezia, Psoriasis, Akne, Dermatitis, oder Onchymykose.

13. Applikations-Vorrichtung, umfassend eine Zubereitung (Z) gemäß einem der Ansprüche 1 bis 12, sowie ein Applikatonssystem (3) für eine Zuführleitung (2) für die Zubereitung (Z), einen Reservoir-behälter für die Zubereitung (Z), eine Treibgasquelle mit Zuführleitung (1) für das Treibgas, und eine Düse (4) zum Versprühen/Verschäumen der Zubereitung (Z).

14. Vorrichtung nach Anspruch 13, **dadurch gekennzeichnet, dass** die Düse (4) eine Vernturi-Düse ist und einen Durchmesser von 0,2 bis 0,8 mm und einen Schaumkopf mit integriertem porösen Körper (Filter) (5) mit Porendurchmesser von 10 bis 50 µm mit Porosität von mehr als 50% aufweist zur Liquid/Gas-Filtration der aerosolierten Zubereitung (Z) und zur Bildung des Schaumproduktes (6).

15. Vorrichtung nach einem der Ansprüche 13 bis 14, **dadurch gekennzeichnet, dass** als poröser Körper ein Gitternetz (Stahl), eine Fritte aus Polyethylen oder ein Vlies aus Viskose oder Polyester vorliegt.

16. Vorrichtung nach einem der Ansprüche 13 bis 15, **dadurch gekennzeichnet, dass** das Treibgas mindestens 25 Vol. % Sauerstoff enthält.

17. Kosmetische Verwendung einer Zubereitung gemäß einem der Ansprüche 1 bis 9 oder 11 zur Anwendung auf Haut/Anhangsgebilde bei Altershaut, trockener, empfindlicher, seborrhoischer Haut, Cellulitis, Vitiligo, Anti-Aging, Rosacea, Allopezia, als UV-Schutz, oder bei allergischer Dermatitis, Akne, Pigmentierungsstörung.
